(19)

**Europäisches Patentamt**

**European Patent Office**

**Office européen des brevets**

(11)　**EP 4 625 417 A1**

(12)　# EUROPEAN PATENT APPLICATION

(43) Date of publication:
**01.10.2025  Bulletin 2025/40**

(21) Application number: **24165826.9**

(22) Date of filing: **25.03.2024**

(51) International Patent Classification (IPC):
**G16B 5/20** *(2019.01)*　　**G16B 40/20** *(2019.01)*
**G16B 10/00** *(2019.01)*

(52) Cooperative Patent Classification (CPC):
**G16B 5/20; G16B 10/00; G16B 40/20**

| | |
|---|---|
| (84) Designated Contracting States:<br>**AL AT BE BG CH CY CZ DE DK EE ES FI FR GB GR HR HU IE IS IT LI LT LU LV MC ME MK MT NL NO PL PT RO RS SE SI SK SM TR**<br>Designated Extension States:<br>**BA**<br>Designated Validation States:<br>**GE KH MA MD TN**<br><br>(71) Applicant: **Julius-Maximilians-Universität Würzburg**<br>**97070 Würzburg (DE)** | (72) Inventors:<br>• **Grün, Dominic**<br>**97228 Rottendorf (DE)**<br>• **Abdelrahman, Mahmoud Aly Mohamed**<br>**97084 Würzburg (DE)**<br><br>(74) Representative: **Eisenführ Speiser**<br>**Patentanwälte Rechtsanwälte PartGmbB**<br>**Gollierstraße 4**<br>**80339 München (DE)** |

(54)　**METHOD FOR GENERATING AN INTERMEDIATE CELLULAR STATE FROM ANCHORING STATES OF A CELLULAR STATE EVOLUTION**

(57)　The present invention relates to a method and to an apparatus for generating at least one intermediate cellular state of a cellular state evolution from an initial cellular state to a corresponding advanced cellular state. The present invention also relates to a computer program for generating at least one intermediate cellular state and to a non-transitory computer readable data medium storing the computer program. Moreover, the present invention relates to a method and to an apparatus for training a model for generating at least one intermediate cellular state of a cellular state evolution from an initial cellular state to a corresponding advanced cellular state. The present invention also relates to a computer program for training a model for generating at least one intermediate cellular state and to a non-transitory computer readable data medium storing the computer program. Furthermore, the present invention relates to a method for diagnosing a disease and forecasting patients' future clinical outcome making use of the method for generating at least one intermediate cellular state. Moreover, the present invention relates to a method for transforming the identity of a cell.

**EP 4 625 417 A1**

**Description**

TECHNICAL FIELD

The present invention relates to a method and to an apparatus for generating at least one intermediate cellular state of a cellular state evolution from an initial cellular state to a corresponding advanced cellular state. The present invention also relates to a computer program for generating at least one intermediate cellular state and to a non-transitory computer readable data medium storing the computer program. Moreover, the present invention relates to a method and to an apparatus for training a model for generating at least one intermediate cellular state of a cellular state evolution from an initial cellular state to a corresponding advanced cellular state. The present invention also relates to a computer program for training a model for generating at least one intermediate cellular state and to a non-transitory computer readable data medium storing the computer program. Furthermore, the present invention relates to a method for diagnosing a disease and forecasting patients' future clinical outcome making use of the method for generating at least one intermediate cellular state. Moreover, the present invention relates to a method for transforming the identity of a cell.

TECHNICAL BACKGROUND

Diseases evolve and manifest due to accumulation of genetic and epigenetic dysregulations over our lifetime. The evolution of diseases is a continuous process and patients usually undergo sequences of certain biological events from a "dysregulated state", which is often undiscovered and not yet accompanied by symptoms, up to the complete manifestation of the disease. These evolutions can be described as "disease-state" trajectories.

Many methods leverage the power of single-cell RNA-sequencing for reconstructing cell state trajectories. Such approaches generally require sufficient sampling of cell states covering the entire trajectory. Since patients typically undergo treatment only after symptoms have already manifested, clinical samples covering intermediate disease states are generally unavailable, limiting the ability to understand the underlying causal paths of disease evolution.

Over the last decade, single cell genomics techniques have been developed which allow describing the regulatory information of several biological systems. Such technologies capture high dimensional data of a cell in a certain state, time point and spatial location.

Computational tools have been established to analyse and describe such snapshot single cell genomics data to define cell types and marker genes associated with underlying cellular states. However, to truly understand the underlying dynamics of a biological system, a densely sampled time-series strategy is needed, which can stitch together the discrete discontinuous snapshots of cellular states to construct the underlying continuum process.

**1 Optimal-Transport approach (Waddington-OT)**

In 2019, the Waddington-OT method was published by (Schiebinger et al., 2019, Cell). In this work, single-cell RNA sequencing was performed for 315,000 cells in a cellular reprogramming experiment, collected at half-day intervals over a period of 18 days.

To infer how these probability distributions evolve over time, the Waddington-OT method uses the mathematical approach of optimal transport (OT) by utilizing scRNA-seq data collected across a time course. Even though the biological process in the experiment is densely sampled over time with half-day intervals, the Waddington-OT method cannot interpolate between cellular states that were sampled with longer time gaps.

**2 RNA velocity approach**

RNA velocity is a computational method that uses single-cell RNA sequencing data to predict the future state of a cell. It is based on the observation that the ratio of unspliced to spliced RNA molecules within a cell provides information about the rate of transcription and splicing, and hence can be used to infer the direction and speed of a cell's differentiation trajectory.

Although RNA velocity can be a useful tool for analysing short-term biological processes (up to a week), it is limited to scenarios of scRNA-seq data without temporal gaps of more than a few hours between cell states. RNA velocity can only predict dynamics on the time-scale of RNA turnover dynamics corresponding to several hours. Larger gaps in the sampling of cell states make it impossible for the method to reconstruct intermediate states. Therefore, if any state is missing, the method may not be able to recover that information. This limitation can be particularly challenging in real-world scenarios where some cell types or states are rare or difficult to obtain.

Additionally, different implementations of RNA velocity have been shown to produce contradicting directionalities for the underlying biological process development (Gorin G et al., 2022, PLoS Comput Biol).

### 3 Variational autoencoders (VAEs) approach

Another tool is the scGen method (Lotfollahi et al, 2019, Nat Methods), which uses variational autoencoders and latent space vector arithmetic to predict perturbation outcomes. Although it was shown that this approach could be useful in modelling simple perturbation and infection responses (one effect at a time), it is limited when predicting complex perturbations or constructing complex intermediate states. This is because scGen, and similar approaches, rely on a simple assumption that arithmetic functions can resolve complex intermediate states in the latent space.

Consequently, with the presently available tools, it is only possible to track the evolution of a (already diagnosed / manifested) disease by collecting and analysing samples over many years of the progression of the disease.

However, for providing sufficient therapeutic options, it is important to interfere already during the evolution of the disease and with the presently available tools, a mechanistic understanding of the underlying causes of such evolving biological systems is missing. More specifically the "intermediate states" are missing which have deep and intimate links to the underlying causal paths of diseases.

## SUMMARY OF THE INVENTION

It is an object of the present invention to provide possibilities for analysing and preferably interfering with the evolution of a disease, particularly preferably before the disease has manifested (e.g. before first symptoms arise).

According to the present invention, a method for generating at least one intermediate cellular state of a cellular state evolution from an initial cellular state to a corresponding advanced cellular state is proposed. The method comprises the steps of:

- providing transcriptome data representing the initial cellular state and the corresponding advanced cellular state,

- providing a model trained by a machine learning for generating the at least one intermediate cellular state on the basis of the initial cellular state and the corresponding advanced cellular state, and

- generating the at least one intermediate cellular state using the provided trained model on the basis of the initial cellular state and the corresponding advanced cellular state.

The invention is based on the recognition that with the known tools mentioned above, it is only possible to analyse and describe a single snapshot in the evolution of a disease. In addition, current single cell analytical approaches are constrained by the observed data and are not capable of reconstructing additional, parallel cellular states or molecular events. Thus, the present tools do not provide (enough) information to gain access to the full continuous process of the evolution of a disease. However, it is particularly the knowledge of the "intermediate states", which provides crucial information, since the disease has not fully manifested yet and the evolution may be slowed, redirected to a less severe direction, or in some cases even be stopped, e.g. by therapeutic treatment. Thus, with information on such "intermediate states", it is possible to screen subjects before a disease has manifested, detect such "intermediate states" in a rather early stage of disease evolution, e.g. before the first symptoms arise, and to interfere with the evolution as described above. Consequently, there is a great need for finding mechanisms to analyse and to interfere with the evolution of a disease, particularly before the disease has manifested (e.g. before first symptoms arise).

It was found that with the method according to the invention, the evolution of diseases can be modelled. As described in the example section, the obtained models were verified, wherein known intermediate states were hidden from the trained model, which however was able to reconstruct these states. Thus, with the method according to the invention, it is possible reconstruct the intermediate disease states, which was a major open challenge in the state of the art.

It is a particular advantage of the method according to the invention that valuable mechanistic insights into the development and progression of diseases at high temporal resolution can be gained.

Preferably, the transcriptome provided in the method is constructed by a DNA microarray, is constructed by a RNA sequencing or is constructed by a single-cell transcriptomics. Particularly preferably, the transcriptome provided in the method is constructed by single cell RNA sequencing.

Preferably, the transcriptome data representing the initial cellular state and the corresponding advanced cellular state are clinical data obtained experimentally from a patient.

It is particularly preferred that the initial and advanced cellular state refer to

- different phases of the cells in cell division,

- different phases of the cells in cellular differentiation,

- different phases of the cells in the transformation of cellular identity, particularly in cellular trans-differentiation,

- different phases of the cells in the transformation of a cell to a cancer cell, or

- different phases of the cells in the transformation of a cancer cell to a metastatic cancer cell.

Preferably, the term "different phases of the cells in cell division" refers to the G0, G1, S, G2 and M phase, wherein the term "different phases" describes that the initial cellular state is selected from the G0, G1, S, G2 and M phase and the advanced cellular state is selected from the G0, G1, S, G2 and M phase but is not the same phase as the initial cellular state.

Preferably, the term "different phases of the cells in cell division" refers to the interphase, prophase, prometaphase, metaphase, anaphase, telophase and the cytokinesis phase, wherein the term "different phases" describes that the initial cellular state is selected from the interphase, prophase, prometaphase, metaphase, anaphase, telophase and the cyto-kinesisphase, and the advanced cellular state is selected from the interphase, prophase, prometaphase, metaphase, anaphase, telophase and the cytokinesis phase but is not the same phase as the initial cellular state.

In case the initial and advanced cellular state refer to different phases of the cells in cell division, the provided transcriptome data representing the initial cellular state and the corresponding advanced cellular state may be from cells in one phase (initial cellular state, e.g. G1 phase or interphase) and from cells in another phase (advanced cellular state, e.g. M phase or telophase). In this case, the at least one intermediate cellular state may be the S phase.

Preferably, the term "cellular differentiation" describes the process in which a stem cell or progenitor cell changes to a cell of less potency, e.g. from totipotent to pluripotent, or multipotent to unipotent. Preferably, the term "cellular differentiation" describes the process in which a stem cell or progenitor cell changes to a differentiated, i.e. mature, cell. During the process of cellular differentiation, several phases are passed. A skilled person knows how to discriminate between the different phases, for example by determining the activity of particular genes. Typically, specific genes are more / less active (measured e.g. on mRNA or protein level) in different phases. Determining the activity of such genes may be applied to determine the phase of cellular differentiation. Additionally or alternatively, a cell may also be assessed for its phase of cellular differentiation by morphological or phenotypical features, e.g. determined by microscopy.

Preferably, the term "cellular identity" describes the type of a cell, preferably wherein the type of a cell may be selected from the group consisting of Brunner's gland cell, Insulated goblet cell, Foveolar cell, Chief cell, Parietal cell, Pancreatic acinar cell, Paneth cell, Type II pneumocyte, Club cell, Type I pneumocyte, Gall bladder epithelial cell, Centroacinar cell, Intestinal brush border cell (with microvilli), K cell, L cell, I cell, G cell, Enterochromaffin cell, Enterochromaffin-like cell, N cell, S cell, D cell, or M cell, Thyroid epithelial cell, Parafollicular cell, Parathyroid chief cell, Oxyphil cell, Alpha cell, Beta cell, Delta cell, Epsilon cell, PP cell (gamma cell), Salivary gland mucous cell, Salivary gland serous cell, Von Ebner's gland cell, Mammary gland cell, Lacrimal gland cell, Ceruminous gland cell, Eccrine sweat gland dark cell, Eccrine sweat gland clear cell, Apocrine sweat gland cell, Gland of Moll cell in eyelid, Sebaceous gland cell, Bowman's gland cell, Corticotropes, Gonadotropes, Lactotropes, Melanotropes, Somatotropes, Thyrotropes, Magnocellular neurosecretory cells, Parvocellular neurosecretory cells, Chromaffin cells (adrenal gland), Keratinocyte, Epidermal basal cell (stem cell), Melanocyte, Trichocyte, Medullary hair shaft cell, Cortical hair shaft cell, Cuticular hair shaft cell, Huxley's layer hair root sheath cell, Henle's layer hair root sheath cell, Outer root sheath hair cell, Surface epithelial cell, basal cell (stem cell), Striated duct cell, Lactiferous duct cell, Ameloblast, Odontoblast, Cementoblast, Auditory inner hair cells of organ of Corti, Auditory outer hair cells of organ of Corti, Basal cells of olfactory epithelium, Cold-sensitive primary sensory neurons, Heat-sensitive primary sensory neurons, Merkel cells of epidermis, Olfactory receptor neurons, Pain-sensitive primary sensory neurons, Photoreceptor rod cells, Photoreceptor blue-sensitive cone cells of eye, Photoreceptor green-sensitive cone cells of eye, Photoreceptor red-sensitive cone cells of eye, Proprioceptive primary sensory neurons, Touch-sensitive primary sensory neurons, Chemoreceptor glomus cells of carotid body cell, Outer hair cells of vestibular system of ear, Inner hair cells of vestibular system of ear, Taste receptor cells of taste bud, Cholinergic neurons, Adrenergic neural cells, Peptidergic neural cells, Inner pillar cells of organ of Corti, Outer pillar cells of the organ of Corti, Inner phalangeal cells of organ of Corti, Outer phalangeal cells of organ of Corti, Border cells of organ of Corti, Hensen's cells of organ of Corti, Vestibular apparatus supporting cells, Taste bud supporting cells, Olfactory epithelium supporting cells, Olfactory ensheathing cells, Schwann cells, Satellite glial cells, Enteric glial cells, Basket cells, Cartwheel cells, Stellate cells, Golgi cells, Granule cells, Lugaro cells, Unipolar brush cells, Martinotti cells, Chandelier cells, Cajal-Retzius cells, Double-bouquet cells, Neurogliaform cells, Retina horizontal cells, Starburst amacrine cells, Renshaw cells, Spindle neurons, Fork neurons, Place cells, Grid cells, Speed cells, Head direction cells, Betz cells, Boundary cells, Bushy cells, Purkinje cells, Medium spiny neurons, Astrocytes, Oligodendrocytes, Tanycytes, Pituicytes, Anterior lens epithelial cell, Crystallin-containing lens fiber cell, White fat cell, Brown fat cell, Liver lipocyte, Cells of the Zona glomerulosa, Cells of the Zona fasciculata, Cells of the Zona reticularis, Theca Interna cell, Corpus luteum cell, Granulosa lutein cells, Theca lutein cells, Leydig cell, Seminal vesicle cell, Prostate gland cell, Bulbourethral gland cell, Bartholin's gland cell, Gland of Littre cell, Uterus endometrium cell, Juxtaglomerular cell, Macula densa cell, Peripolar cell, Mesangial cell, Parietal epithelial cell, Podocyte, Proximal tubule brush border cell, Loop of Henle thin segment cell, Kidney distal tubule cell, Principal cell, Intercalated cell, Transitional epithelium, Duct cell, Efferent ducts cell, Epididymal principal cell, Epididymal basal cell, Endothelial cells, Planum semilunar epithelial cell of vestibular system of ear, Organ of Corti interdental epithelial cell, Loose connective tissue fibroblasts, Corneal fibroblasts, Tendon fibroblasts, Bone marrow reticular tissue fibroblasts, Other nonepithelial fibroblasts, Hepatic stellate cell (Ito cell), Nucleus pulposus cell, Hyaline cartilage chondrocyte, Fibrocartilage chondrocyte, Elastic cartilage chondrocyte, Osteoblast/osteocyte, Osteoprogenitor cell, Hyalocyte, Stellate cell, Pancreatic stellate cell, Red skeletal muscle cell (slow twitch), White skeletal muscle cell (fast twitch), Intermediate skeletal muscle cell, Nuclear bag cell, Nuclear chain cell, Myosatellite cell (stem cell), Cardiac muscle cell, SA node cell, Purkinje fiber cell, Smooth muscle cell, Myoepithelial cell, Erythrocyte, Megakaryocyte, Platelets if considered distinct cells, currently there's debate on the subject., Monocyte, Connective tissue macrophage, Epidermal Langerhans cell, Osteoclast, Dendritic cell, Microglial cell, Neutrophil granulocyte, Eosinophil granulocyte and precursors, Basophil granulocyte and precursors, Mast cell, Helper T cell, Regulatory T cell, Cytotoxic T cell, Natural killer T cell, B cell, Plasma cell, Natural killer cell, Hematopoietic stem cells and committed progenitors for the blood and immune system, Oogonium/Oocyte, Spermatid, Spermatocyte, Spermatogonium cell, Spermatozoon, Granulosa cell, Sertoli cell, Epithelial reticular cell and Interstitial kidney cells.

In case the initial and advanced cellular state refer to different phases of the cells in cellular differentiation, the provided transcriptome data representing the initial cellular state and the corresponding advanced cellular state may be from cells of particular potency (initial cellular state, e.g. multipotency) and from cells of less potency than in the initial cellular state (advanced cellular state, e.g. oligopotency or unipotency). In this case, the at least one intermediate cellular state may be a cell of less or equal potency than in the initial cellular state but of higher or equal potency than in the advanced cellular state.

Preferably, the provided transcriptome data representing the initial cellular state and the corresponding advanced cellular state may be from cells of different potency during hematopoiesis.

Preferably, the term "cellular transdifferentiation" describes the process in which one mature somatic cell is transformed into another mature somatic cell without undergoing an intermediate pluripotent state or progenitor cell type. For example, alpha-cells in the pancreas may transdifferentiate to beta-cells in the pancreas, as reported in humans. Further, the cellular transdifferentiation may occur spontaneously or may be induced. A skilled person knows how to discriminate cell types, for example by determining the activity of particular genes and/or by assessing morphological or phenotypical features, e.g. determined by microscopy.

In case the initial and advanced cellular state refer to different phases of the cells in the transformation of cellular identity, the provided transcriptome data representing the initial cellular state and the corresponding advanced cellular state may be from particular mature somatic cells (initial cellular state, e.g. epithelial cells or fibroblasts) and from particular other mature somatic cells (advanced cellular state, e.g. mesenchymal cells or cardiomyocytes). In this case, the at least one intermediate cellular state may be a cell in the process of transformation from epithelial identity to mesenchymal identity or from fibroblasts to cardiomyocytes.

Preferably, the term "transformation of a cell to a cancer cell" refers to carcinogenesis. Preferably, the term describes a process, in which a cell undergoes changes at the cellular, genetic, and/or epigenetic level and/or shows abnormal cell division. A skilled person knows how to identify a cancer cell, for example by determining the activity of particular genes and/or by assessing morphological or phenotypical features, e.g. determined by microscopy.

In case the initial and advanced cellular state refer to different phases of the cells in the transformation of a cell to a cancer cell, the provided transcriptome data representing the initial cellular state and the corresponding advanced cellular state may be from healthy cells (initial cellular state, e.g. plasma cells) and from cancer cells (advanced cellular state, e.g. myeloma cells). In this case, the at least one intermediate cellular state may be a cell of higher malignancy than the initial cellular state but lower malignancy than the advanced cellular state, for example MGUS (monoclonal gammopathy of undetermined significance) cells or smoldering myeloma cells.

Preferably, the term "transformation of a cancer cell to a metastatic cancer cell" describes a process, in which a cancer cell (or a cancerous tumour) gains the ability to spread from the primary cancer site to a secondary cancer site. A skilled person knows how to identify a metastatic cancer cell, for example by determining the cell type of the cancer cell and assessing whether this cell type is typically found at the cancer site, where it was located.

In case the initial and advanced cellular state refer to different phases of the cells in the transformation of a cancer cell to a metastatic cancer cell, the provided transcriptome data representing the initial cellular state and the corresponding advanced cellular state may be from non-metastatic cancer cells (initial cellular state), and from metastatic cancer cells (advanced cellular state). In this case, the at least one intermediate cellular state may be a cell in the process of transformation from a cancer cell to a metastatic cancer cell.

Additionally or alternatively, based on the at least one intermediate cellular state, a causal trajectory for of the cellular state evolution from the initial cellular state to the corresponding advanced cellular state is generated.

The model trained by machine learning may be a classification model such as a neural network that is trained for generating at least one intermediate cellular state of a cellular state evolution from an initial cellular state to a corresponding advanced cellular state. The neural network may be trained using transcriptome training data.

In particular, the model trained by machine learning may have a deep reinforcement learning architecture where a neural network is trained for generating at least one intermediate cellular state of a cellular state evolution trajectory from an initial cellular state to a corresponding advanced cellular state. The neural network may be trained using single cell transcriptomic data. The deep reinforcement learning architecture preferably implements a policy network, which is parametrized by a multilayer perceptron (MLP) and which may be optimized via gradient ascent to maximize the reward objective. In addition to the standard reward objective, the policy network may be configured to optimize a maximum entropy objective to enable the model to alternate between exploration and exploitation phases during reconstruction.

Optionally, the model is trained by a deep reinforcement learning technique. For example, the trained model may be trained, e.g., by deep reinforcement learning, as described below using the method or the apparatus for training a model by machine learning for generating at least one intermediate cellular state.

The present invention also relates to a computer program configured for generating at least one intermediate cellular state of a cellular state evolution from an initial cellular state to a corresponding advanced cellular state, the computer program including instructions for executing the steps of the method for generating at least one intermediate cellular state described before, when run on a computer. The present invention also relates to a non-transitory computer readable data medium storing the computer program for generating at least one intermediate cellular state.

The present invention also relates to an apparatus for generating at least one intermediate cellular state of a cellular state evolution from an initial cellular state to a corresponding advanced cellular state. The apparatus comprises a transcriptome data providing unit, a trained model providing unit and an intermediate cellular state generating unit. The transcriptome data providing unit is configured for providing transcriptome data representing the initial cellular state and the corresponding advanced cellular state. The trained model providing unit is configured for providing a model trained by a machine learning for generating the at least one intermediate cellular state on the basis of the initial cellular state and the corresponding advanced cellular state. The intermediate cellular state generating unit is configured for generating the at least one intermediate cellular state using the provided trained model on the basis of the initial cellular state and the corresponding advanced cellular state. The apparatus can be used for carrying out the method for generating at least one intermediate cellular state described before.

According to the present invention, also a method for training a model by machine learning is proposed. The method comprises the steps of:

i) providing a plurality of transcriptome training data, each representing an initial cellular state and a corresponding advanced cellular state of a cellular state evolution,

ii) providing a model that can be trained by machine learning, and

iii) training the model by machine learning using the transcriptome training data such that the trained model is configured to receive transcriptome data representing an initial cellular state and a corresponding advanced cellular state of a cellular state evolution and for generating at least one intermediate cellular state of the cellular state evolution from the initial cellular state to the corresponding advanced cellular state based on the received transcriptome data.

Preferably, the transcriptome training data, each representing the initial cellular state and the corresponding advanced cellular state, respectively, are clinical data obtained experimentally from one or more patients. In the training method according to the invention, the to-be-trained model may generate actions in gene expression space and may learn stochastic policies to reconstruct trajectories connecting two distant anchoring cellular states, i.e., an initial cellular state to the corresponding advanced cellular state of a cellular state evolution.

It is preferred in the training method according to the invention that step iii) comprises at least one of the steps:

iii.1) generating transcriptome data of further cellular states by sampling from a multivariate Gaussian distribution to learn combinations of action parameters comprising a control mean and a standard deviation of the generated transcriptome data of transcriptome data of further cellular states, e.g., using a Gen-unit,

iii.2) classifying the generated transcriptome data, e.g., from the Gen-unit, of further cellular states based on the learned combinations of action parameters to identify those combinations of action parameters that fulfil a predefined reward criterion, e.g., using a Do unit,

iii.3) calculating a distance of the generated transcriptome data of further cellular states to the transcriptome data of advanced cellular states of the plurality of transcriptome training data, e.g., using a distance calculating unit,

iii.4) rewarding the generated transcriptome data of further cellular states with reference to a predefined reward criterion, e.g., using a rewarding unit, and

iii.5) selecting or emitting a combination of action parameters to generate transcriptome data of further cellular states, which have the highest probability of an increasing reward from one generated transcriptome data to the next transcriptome data within the sequence, as the transcriptome data of a plurality of sequential intermediate cellular states, e.g., using an action parameters selecting unit. For example, the action parameters selecting unit may comprise a policy network. In particular, step iii.5) may be implemented in that the policy network emits a combination of action parameters to generate transcriptome data of further cellular states, which have the highest probability of an increasing reward from one generated transcriptome data to the next transcriptome data within the sequence, as the transcriptome data of a plurality of sequential intermediate cellular states, e.g., using an action parameters from the trained policy network.

It is further preferred in the method according to the invention that the advanced cellular state in step i) is divided into a plurality of groups of different severity of the advanced cellular state,
preferably wherein the transcriptome data of a plurality of sequential intermediate cellular states selected and generated in step iii.5) is assigned to one of the groups of different severity of the advanced cellular state.
The groups of different severity may be assigned in any way, for example the different severities are ranked on a numerical scale.
It is further preferred in the method according to the invention that step iii) further comprises
iii.6) repeating the steps iii.1) to iii.5) to generate a plurality of transcriptome data of a plurality of sequential intermediate cellular states.
According to the present invention, also an apparatus for training a model by machine learning is proposed. The apparatus comprises a transcriptome training data providing unit, a model-to-be-trained providing unit and a model training unit. The transcriptome training data providing unit being configured for providing a plurality of transcriptome training data, each representing an initial cellular state and a corresponding advanced cellular state of a cellular state evolution. The model-to-be-trained providing unit being configured for providing a model that can be trained by machine learning. The model training unit being configured for training the model by machine learning using the transcriptome training data such that the trained model is configured to receive transcriptome data representing an initial cellular state and a corresponding advanced cellular state of a cellular state evolution and for generating at least one intermediate cellular state of the cellular state evolution from the initial cellular state to the corresponding advanced cellular state based on the received transcriptome data. With the training apparatus it is possible to carry out the method for training a model by machine learning for generating at least one intermediate cellular state described herein.
Preferably, the model training unit comprises at least one of the following:

- a Gen-unit that is configured for generating transcriptome data of further cellular states by sampling from a multivariate Gaussian distribution to learn combinations of action parameters comprising a control mean and a standard deviation of the generated transcriptome data of transcriptome data of further cellular states,

- a Do-unit that is configured for applying the actions parameters to the Gen-unit to generate novel transcriptome data of further cellular states.

- a distance calculating unit that is configured for calculating a distance of the generated transcriptome data of further cellular states to the transcriptome data of advanced cellular states of the plurality of transcriptome training data,

- a rewarding unit that is configured for rewarding the generated transcriptome data of further cellular states with reference to a predefined reward criterion, and

- an action parameters selecting unit that is configured for selecting a combination of action parameters to generate transcriptome data of further cellular states, which have the highest probability of an increasing reward from one generated transcriptome data to the next transcriptome data within the sequence, as the transcriptome data of a plurality of sequential intermediate cellular states.

In particular, the action parameters selecting unit may comprise a policy network that is trained for selecting a combination of action parameters to generate transcriptome data of further cellular states, which have the highest probability of an increasing reward from one generated transcriptome data to the next transcriptome data within the sequence, as the transcriptome data of a plurality of sequential intermediate cellular states.
A possible exemplary implementation of the training apparatus will be explained in the following:

For example, it is possible that a given cellular state may be represented as $s \in \mathbb{R}x$, with cell index $x = 1, \ldots, C$ and gene index $j = 1, \ldots, G$. The initial cellular state may be denoted as $Si \in \mathbb{R}x$, and the corresponding advanced cellular state of interest as $Sf \in \mathbb{R}x,j$. It is possible to generate a sequence of intermediate cellular states $Sgen \in \mathbb{R}x$, representing a causal path connecting two anchoring cellular states ($Si$, $Sf$).

To train a model in order to be able to find the missing cellular state **Sgen**, the training apparatus may comprise the following components: an action generator and controller **(AGC)** Unit, an observation space **(O)**, a reward function **(R)**, and a policy network ($\pi\theta$).

The environment may be designed to be represented as an infinite-horizon Markov decision process (MDP). The MDP may be defined by a tuple (**S**, **A**, **r**), where **S** represents a complete description of the environment state and **A** represents a continuous action space in the environment. The transition probability **P** represents the probability density of the next state **st+1** $\in$ **S** given the current state of the environment **st** $\in$ **S** and the action **at** $\in$ **A.**

A trajectory $\tau$ is defined as a sequence of states and actions in the environment through **H** steps:

$$\tau H = (s_0, a_0, s_1, a_1, \ldots, s_H, a_H)$$

The transition from one cellular state to another is governed by a stochastic state transition function:

$$s_{t+1} \sim P(\cdot \mid s_t, a_t)$$

The actions are generated by the model according to the policy network $\pi\theta$ which is parametrized by :

$$a_t \sim \pi\theta(\cdot \mid s_t)$$

The environment may release reward **rt** after each transition which is dependent on the current state of the environment, the actions taken, and the environment's next state given the reward function **R:**

$$r_t = R\,(s, a, s_{t+1})$$

Preferably, it is possible to find the parameters $\theta$ of the optimum policy $\pi\theta$ (**at** | **st**) by maximizing the expected total reward ($\pi$) summed over the trajectory,

$$J(\pi) = \max_{\pi} \mathrm{E}\left[\sum_{t=0}^{H} \gamma^t R(S_t, A_t, S_{t+1}) \mid \pi\right]$$

where the horizon parameter **H** represents the number of steps on each trajectory:

$$\pi_\theta = \underset{\pi}{argmax}\, J(\pi)$$

The reward objective may be extended to an infinite-horizon discounted return ($\tau$) over a trajectory, with a discounting factor $\gamma \in$ (**0, 1**):

$$R(\tau) = \sum_{t=0}^{\infty} \gamma^t r_t$$

where $\gamma$ **< 1** induces a discount effect on the overall return.

**Actions Generator and Controller (AGC) unit:**

The AGC may be part of the model training unknit and may be composed of two sub-units, the Gen-Unit mentioned with reference to step iii.1 of the training method according to the invention and the Do-Unit mentioned with reference to step iii.2 of the training method according to the invention.

**I. Gen-Unit:**

The Gen-Unit that preferably is part of the model training unit may be configured to generate cellular states in the gene expression space.

The generation process may start with concatenating *si* and *sf* into a matrix $M \in \mathbb{R}x$ , :

$$M = [s_i \ s_f]$$

The M matrix may be pushed to a standard data pre-processing unit to perform log-normalization (using size factor $10^4$ molecules for each cell) and to scale the gene expression values for each gene (z-score transformation). The AGC unit may transpose the pre-processed M and calculates the gene-gene covariance matrix $\Sigma \in \mathbb{R}j$ ,:

$$\Sigma = cov\,(M^T)$$

To extract the orthogonal axes of gene expression variability characteristic of the gene regulatory networks governing *si* and *sf*, the AGC may perform singular value decomposition (SVD) on $\Sigma$,

$$\Sigma = U \Lambda V^T$$

where

a) $U \in \mathbb{R}$ .,/ represents the left-singular vectors of $\Sigma$ with **f** decomposed factors,

b) $\Lambda \in \mathbb{R}$ .,. represents the singular-value diagonal matrix, and

c) $VT \in \mathbb{R}$ /,. represents the right-singular vectors of $\Sigma$.

The Gen-Unit may be configured to generate new cellular states **sgen** by sampling from a multivariate Gaussian distribution $X \in \mathbb{R}^{xgen,f}$ ,

$$X \sim \mathcal{N}\,(\mu_{gen,}\ \sigma_{gen})$$

where the two parameters $\mu gen$ and $\sigma gen$ control mean and standard deviation of the generated cellular state **sgen** and each dimension implements an action on one of the factors. The chosen dimension *xgen* controls the number of generated cells.

$$\Psi = U.\sqrt{\Lambda + \zeta}$$

$$s_{gen} = X\,.\Psi^T + \frac{\mu_j}{\max|\mu_j|}$$

Since **U** describes the direction of $\Sigma$ matrix's underlying maximum action, $\zeta$ may be introduced as a scaling parameter for $\Lambda$, to control the action magnitude. Finally, $\mu j$ represents the mean gene expression of the two cellular states **M,** which is "max" normalized in **sgen.**

**II. Do-unit:**

The Do-unit that preferably is part of the model training unit may optionally be introduced for controlling the generated cellular states in the gene expression space via do operation on the ($\mu gen,$ $\sigma gen$ and $\zeta$) Gen-Unit parameters,

$$c_{gn} \sim \mathbf{do}\ (\mu_{gen} = 0,\ \sigma_{gen} > 0)$$

$$c_{oe} \sim \mathbf{do}\ (\mu_{gen} > 0,\ \sigma_{gen} > 0)$$

$$c_{kd} \sim \mathbf{do}\ (\mu_{gen} < 0,\ \sigma_{gen} > 0)$$

$$Pert_{sv} \sim \mathbf{do}\ (\zeta > 0)$$

where **cgn**, **coe** or **ckd** represent possible outcomes of the Do-unit, i.e., Gaussian noise, overexpression, or knockdown, respectively, for the newly generated cellular state sgen. In addition, *Pertsv* may be sued to perturb the singular values by interventions through tuning the scaling parameter $\zeta$.

It is desirable to find the controller parameters ($\mu_{gen}$, $\sigma_{gen}$ and ) of optimum policy. Ultimately, the optimum policy may guide the model-to-be-trained to provide a sequence of actions and to reconstruct $\mathbf{S_{gen}}$ in **H** steps for a given $\tau_H$.

In the following, the observation space is introduced and it is outlined how the environment is observed.

**The Observation Space:**

The observation space preferably is a cellular state manifold representing all possible configurations of a cell. Certain regions of interest within this space can be visited and reconstructed. Such regions of the observation space are termed Reconstruction Field (RF).

To enable gaining a sense of directionality and distances in the RF, one or more of a state observation (**O**), a cellular states distance (CSD), learning bound scores (LBS), and a cellular state loss (CSL) may be used.

**I. Single Observation (O):**

A given cellular state **s** may be summarized into a single observation **O** according to the following equation,

$$O = SoftMax\ (MGA\ (s))$$

where the median gene activity (MGA) of a given cellular state **s** is calculated and rescaled via SoftMax function to *j-dimensional* output ranging from [0,1] and is summing to 1.

**II. Cellular States Distance (CSD):**

First, a Cellular States Distance (CSD) as mentioned in the iii.3 of the training method between the initial cellular state observation **O_i** and the target cellular observation **O_f** may be calculated

$$CSD\ (O_i |\ O_f) = \left(1 - S_{cos}(O_i |\ O_f)\right) \times \vartheta\ D_{KL}(O_i \| \ O_f)$$

The **CSD** is composed of two terms:

    **a)** Cosine similarity term: $1 - S_{cos}\ (O_i \| O_f)$
    **b)** Kullback-Leibler divergence term: $D_{KL}(O_i \| O_f)$

The parameter $\vartheta$ defines the importance of the $D_{KL}$ terms to the **CSD**.

**III. Learning bound scores (LBS)**

The LBS preferably define the boundaries of the cellular state reconstruction field, comprising one or more of the following cellular bound scores:

$$RBmin = CSD \times \alpha,$$

$$RBmax = CSD \times \beta,$$

$$TBmax = CSD \times \gamma,$$

$$TBmin = \omega$$

where $\alpha$ controls the minimum reward bound **RBmin**, $\beta$ controls the maximum reward bound **RBmax**, $\gamma$ controls the maximum trajectory bound **TBmax** and $\omega$ controls the minimum trajectory bound **TBmin.**

### IV. Cellular State loss (CSL):

The cellular state loss (CSL) may be employed to enable the model to learn the distances between generated and target cellular state observations:

$$CSL\left(O_{gen} \mid O_f\right) = \left(1 - S_{cos}(O_{gen} \mid O_f)\right) \times \vartheta \, D_{KL}\left(O_{gen} \parallel O_f\right)$$
$$- \lambda \sum P(O_{gen}) \times log\left(P(O_{gen})\right)$$

The CSL may be composed of one or more of the following terms:

    **a)** Cosine similarity term: $1 - S_{cos}(O_i \parallel O_f)$
    **b)** Kullback-Leibler divergence term: $D_{KL}(O_i \parallel O_f)$
    **c)** Entropy regularization term

**Ogen** and **Of** represent a generated observation and a target state observation, respectively. The $\vartheta$ and $\lambda$ parameters define the weights of the $D_{KL}$ and entropy regulation terms.

### Reward Function:

The reward function of the environment as mentioned in step iii.4 of the training method according to the present invention may depend on the current cellular state observations and the **CSL** incurred by the newly generated cellular state,

$$R\left(s, a, s_{t+1}\right) = \rho + (1 - CSL)^a$$

where $\rho$ is defining the reward signal and $\alpha$ is the reward coefficient. The model may be conditioned get *positive or negative* rewards given the **CSL**:

$$R\left(s, a, s_{t+1}\right) = \begin{cases} r_t = \rho + (1 - CSL)^\alpha, & if \, CSL \geq RB_{min} \wedge CSL \leq RB_{max} \\ r_t = -1, & otherwise \end{cases}$$

If the **CSL** is between **RBmin and RBmax**, such behavior is reinforced, and a *positive rt* is received. In contrast, if the reward bounds are exceeded, a *negative rt* is received.

Furthermore, a termination condition **T** with a **done** signal may be introduced to the environment, which depends on $TB_{max}$ and $TB_{min}$ as well as the number of steps, for a given cellular state trajectory $\tau_H$. **n** starts at **n = (H** - 1) and is decreased by one at each step:

$$T\left(CSL\right) = \begin{cases} done = True, & if \, CSL \geq TB_{max} \\ done = True, & if \, CSL \leq TB_{min} \\ done = True, & if \, n \leq 0 \\ done = False, & otherwise \end{cases}$$

**Policy Network:**

The policy $\pi_\theta$ preferably is parametrized by a neural network and optimized via gradient ascent, where the $\alpha$ parameter controls the learning rate:

$$\theta \leftarrow \theta + \alpha \nabla_\theta \mathbb{E}_{\pi\theta} R(\tau)$$

The gradient may be used to *increase* the probability of paths with *positive* $r_t$ values and *decrease* the probability of paths with *negative* $r_t$ values. Monte Carlo sampling of **N** trajectories may be performed and the policy $\pi_\theta$ may be run. Then, a multilayer perceptron (MLP) may be fit to estimate the return and to improve the policy iteratively:

$$\nabla_\theta J(\theta) \approx \frac{1}{N} \sum_{i=1}^{N} \left( \sum_{t=1}^{H} \nabla_\theta \log \pi_\theta(a_{i,t} \mid s_{i,t}) \right) \left( \sum_{t=1}^{H} r(s_{i,t}, a_{i,t}) \right)$$

In addition to optimizing for the standard maximum reward objective, the Soft-Actor-Critic maximum entropy objective may be considered:

$$\mathcal{H}\left(\pi(.\mid s_t)\right) = \mathbb{E}_{a \sim \pi(\cdot \mid s)}[-\log\left(\pi(a \mid s)\right)]$$

The entropy term $\mathcal{H}$ controls the stochastic behavior of model policies $\pi_\theta$. The maximum entropy objective enables the model to alternate between exploration and exploitation phases during training:

$$\pi_\theta = \underset{\pi}{argmax} \, \mathbb{E}_{\tau \sim \pi} \left[ \sum_{t=0}^{\infty} \gamma^t \left( R(s_t, a_t, s_{t+1}) + \alpha \mathcal{H}(\pi(\cdot \mid s_t)) \right) \right]$$

The model-to-be-trained may get rewards proportional to the entropy term $\mathcal{H}$ in the policy $\pi_\theta$, and the $\alpha$ parameter determines the relative importance of the $\mathcal{H}$ given the reward at each time step. Higher $\alpha$ corresponds to more exploration and lower $\alpha$ corresponds to more exploitation.

**Optimal Causal Paths (OCP):**

To define the causal paths given the predicted actions, the optimum causal paths (OCP) concept may be employed. The model to-be-trained may generate actions from the trained policy network $\pi_\theta$. The predicted action parameters $\mu_{gen}$, $\sigma_{gen}$ and $\zeta$ values may be pushed to the Gen-Unit. The **CSL** may be calculated for each path $\tau_H$ and a matrix with **k** sampled realizations and cellular state losses $P^{k,n}$ may be constructed accordingly:

$$P^{k,n} = CSL\,(G, a_t)$$

The trained model may generate **k** realizations representing generated paths with **H** steps where each path connects the same two anchoring cellular states (**S**, **Sf**). Ultimately, to define the optimal paths $OCP_i$, the quantiles $Q_j$ maybe calculated for the $P^{k,}$ matrix,

$$OCP_i(S_{gen}, S_f) = Q_j\,(P^{k,n}, j)$$

where **0 <= j <= 1** to stratify paths according to the **CSL** distribution:

$$OCP_i(S_{gen}, S_f) = \begin{cases} \text{path} = \text{Min}, & \text{if } j = 0.1 \\ \text{path} = \text{Optimum}, & \text{if } j = 0.5 \\ \text{path} = \text{Max}, & \text{if } j = 0.9 \\ \text{path} = \text{Other}, & \text{otherwise} \end{cases}$$

The present invention also relates to a computer program configured for training a model by machine learning to generate at least one intermediate cellular state of a cellular state evolution from an initial cellular state to a corresponding advanced cellular state, the computer program including instructions for executing the steps of the method for training a model by machine learning, when run on a computer. The present invention also relates to a non-transitory computer readable data medium storing the computer program for training a model by machine learning.

The present invention further relates to a method for diagnosing a disease, comprising

a) providing a sample of a subject, wherein the sample comprises cells associated with the disease to be diagnosed,

b) obtaining the transcriptome data of one or more cells from the sample obtained in step a), and

c) comparing the transcriptome data obtained in step b) with the reconstructed transcriptome data of the plurality of sequential intermediate cellular states as generated in the method for generating at least one intermediate cellular state according to the present invention,

wherein the disease is diagnosed if the transcriptome data obtained in step b) corresponds to one of the transcriptome data of the plurality of sequential intermediate cellular states.

Preferably, the term "disease" refers to any disease. Preferably, the term "disease" refers to a disease selected from the group consisting of autoimmune diseases, cancerous diseases, genetic diseases, infectious diseases, inflammatory diseases, neurodegenerative diseases.

Preferably, the term "cells associated with the disease to be diagnosed" describes cells of a cell type, which is typically associated with the disease to be diagnosed.

It is a particular advantage of the present invention that a plurality of sequential intermediate cellular states is generated, which are associated with a state in the evolution of a disease.

Thus, in case of a subject, which does not (yet) suffer from symptoms of a disease, the transcriptome data corresponds to the transcriptome data of an intermediate cellular state as described herein, a diagnosis of the disease may be made. The diagnosis may therefore be made before (even long before) first symptoms arise, for example in case the sample of the subject is provided in step a) during a routine check-up or during an examination due to the presence of one or more risk factors for a particular disease.

For almost all diseases known today, the chances for preventing or lessening the disease or for slowing down its evolution and/or progression are dramatically increased in case a diagnosis is made as early as possible. With the method according to the invention, it is possible to diagnose a disease (long) before first symptoms arise and thus long time before a subject usually asks for medical examination.

It is further advantageous that for the diagnosis of the disease, only a sample of a subject needs to be provided. After providing a sample, no action or interaction of the subject is required for making the diagnosis.

The term "corresponds" as used in the method according to the invention describes an overlap or a complete match. Preferably, the term "corresponds" describes an identity of the transcriptomes of at least 95 %, preferably at least 96 %, preferably at least 97 %, preferably at least 98 %, preferably at least 99 %, preferably at least 99.5 %, preferably 100 %.

Preferably, the term "identity of the transcriptomes" describes that at least 95 %, preferably at least 96 %, preferably at least 97 %, preferably at least 98 %, preferably at least 99 %, preferably at least 99.5 %, preferably 100 % of the detected transcripts of one transcriptome are present in the other transcriptome. Preferably, the respective quantity of these transcripts, which are present in both transcriptomes, does not differ more than 10 %, preferably not more than 7.5 %, preferably not more than 5 %, preferably not more than 2.5 %, preferably wherein the higher quantity of a transcript is set to 100 % for the comparison.

Preferably, the term "transcriptome" refers to all RNA in a sample, particularly preferably only to the mRNA in a sample.

It is further preferred in the method according to the invention that in step c) the transcriptome data obtained in step b) is further compared with the transcriptome data of a plurality of cells in an advanced cellular state as provided in step i) of the method according to the invention, and/or with the transcriptome data of a plurality of cells in an initial cellular state as provided in step i) of the method according to the invention,

wherein the disease is diagnosed if the transcriptome data obtained in step b) corresponds to one of the transcriptome data of the plurality of sequential intermediate cellular states or to one of the transcriptome data of the advanced cellular state,
and/or

wherein the disease is not diagnosed if the transcriptome data obtained in step b) corresponds to one of the transcriptome data of the initial cellular state.

Thus, in case the transcriptome of a sample corresponds to the initial cellular state, it is preferably assumed that the disease does not (yet) evolve in the subject of which the sample was provided.

In contrast, in case the transcriptome of a sample corresponds to the advanced cellular state, it is preferably assumed that the disease has already evolved in the subject of which the sample was provided.

It is preferred in the method for diagnosing a disease according to the invention that the disease is cancer, preferably metastasizing cancer.

Preferably, the term "metastasizing cancer" refers to cancer, wherein one or more cancer cell(s) has/have gained the ability to spread from the primary cancer site to a secondary cancer site. A skilled person knows how to identify metastasizing cancer, for example by determining the cell type of a cancer cell and assessing whether this cell type is typically found at the cancer site, where it was located.

What was said with regard to the method for providing at least one intermediate cellular state of a cellular state evolution from an initial cellular state to a corresponding advanced cellular state applies accordingly to the method for diagnosing a disease.

The present invention further relates to a method for predicting disease progression, comprising

A) providing a sample of a subject, wherein the sample comprises cells associated with the disease to be diagnosed,

B) obtaining the transcriptome data of one or more cells from the sample obtained in step A), and

C) comparing the transcriptome data obtained in step B) with the reconstructed transcriptome data of the plurality of sequential intermediate cellular states as generated in the method for generating at least one intermediate cellular state according to the present invention,

to identify one of the transcriptome data of the plurality of sequential intermediate cellular state as generated in the method according to the invention, to which the transcriptome data obtained in step B) corresponds

wherein the prediction of the disease is based on the group of the severity of the disease, to which the transcriptome data identified in step C) is assigned.

In addition to the advantages described above, it is a particular advantage of the present invention that groups of different severity of the disease may be formed. In step C) of the method for predicting disease progression according to the present invention, it is thus possible to assign the transcriptome in a sample to a particular group, which is assigned to a particular disease severity. The groups of different severity may be assigned in any way, for example the different severities are ranked on a numerical scale.

Preferably, the term "predicting disease progression" refers to the severity of the disease.

The term "corresponds" as used in the method according to the invention describes an overlap or a complete match. Preferably, the term "corresponds" describes an identity of the transcriptomes of at least 95 %, preferably at least 96 %, preferably at least 97 %, preferably at least 98 %, preferably at least 99 %, preferably at least 99.5 %, preferably 100 %.

Preferably, the term "identity of the transcriptomes" describes that at least 95 %, preferably at least 96 %, preferably at least 97 %, preferably at least 98 %, preferably at least 99 %, preferably at least 99.5 %, preferably 100 % of the detected transcripts of one transcriptome are present in the other transcriptome. Preferably, the respective quantity of these transcripts, which are present in both transcriptomes, does not differ more than 10 %, preferably not more than 7.5 %, preferably not more than 5 %, preferably not more than 2.5 %, preferably wherein the higher quantity of a transcript is set to 100 % for the comparison.

Preferably, the term "transcriptome" refers to all RNA in a sample, particularly preferably only to the mRNA in a sample.

What was said with regard to the method for providing at least one intermediate cellular state of a cellular state evolution from an initial cellular state to a corresponding advanced cellular state or with regard to the method for diagnosing a disease applies accordingly to the method for predicting disease progression.

It is preferred in the method for predicting disease progression according to the invention that the disease is cancer, preferably metastasizing cancer.

Preferably, the term "metastasizing cancer" refers to cancer, wherein one or more cancer cell(s) has/have gained the ability to spread from the primary cancer site to a secondary cancer site. A skilled person knows how to determine metastasizing cancer, for example by determining the cell type of a cancer cell and assessing whether this cell type is typically found at the cancer site, where it was located.

The present invention further relates to a method for transforming the identity of a cell, the method comprising

1) generating at least one intermediate cellular state of a cellular state evolution from an initial cellular state to a corresponding advanced cellular state, with a method according to the invention,
wherein the initial and advanced cellular state refer to different phases of the cells in the transformation of cellular identity, particularly in cellular transdifferentiation,

2) analysing the at least one intermediate cellular state generated in step 1) to identify a cellular signalling pathway involved in the transformation of the cellular identity,

3) modulating, preferably activating or inactivating, the cellular signalling pathway identified in step 2) to promote the transformation of the identity of a cell.

Preferably, for identifying a cellular signalling pathway involved in the transformation of the cellular identity, the generated intermediate cellular state is compared with the initial cellular state. Statistically significant changes in e.g. the protein level or the phosphorylation of a protein between the generated intermediate cellular state and the initial cellular state are preferably considered as being involved in the transformation of the cellular identity. Thus, a cellular signalling pathway including the e.g. synthesis or phosphorylation of said protein is considered as a cellular signalling pathway involved in the transformation of the cellular identity.

Preferably, the method for transforming the identity of a cell is a non-therapeutic method.

Preferably, the method for transforming the identity of a cell is an in-vitro method.

BRIEF DESCRIPTION OF THE DRAWINGS

Fig. 1A: shows an overview of a model environment architecture where blue dots (1) represent the initial cellular state, orange dots (2) represent the generated cellular states after the model has taken actions, and the red dots (3) represent the corresponding advanced cellular states;

Fig. 1B: shows a schematic representation of an observation space of healthy and disease cellular states (right), where each dot represents a real data point collected from experimental and clinical data. By zooming into the data of the two states in the small box, it is focused on the reconstruction field of interest given the initial and corresponding advanced states of interest. The model, through trial and error, tries to generate cellular states and trajectories in the reconstruction area, approximating the true cellular state manifold;

Figs. 1C,D: show a schematic representation of the learning bound scores. By providing initial and corresponding advanced states, it is possible able to define the reconstruction area of interest. It is possible also define the positive reward area, where the model is encouraged to take actions and generate cellular states within that area. Conversely, the negative reward area is defined to discourage the model from generating cellular states in that region;

Fig. 1E: shows an action generator and a controller unit, where the covariance matrix of the initial and corresponding advanced cellular states is decomposed using the SVD algorithm. Subsequently, the singular value and vector matrices are fed into a Gen-Unit to generate novel cellular states. The generator unit is controlled by the Do-Unit, which the model uses to manage the generation parameters of the Gen-Unit;

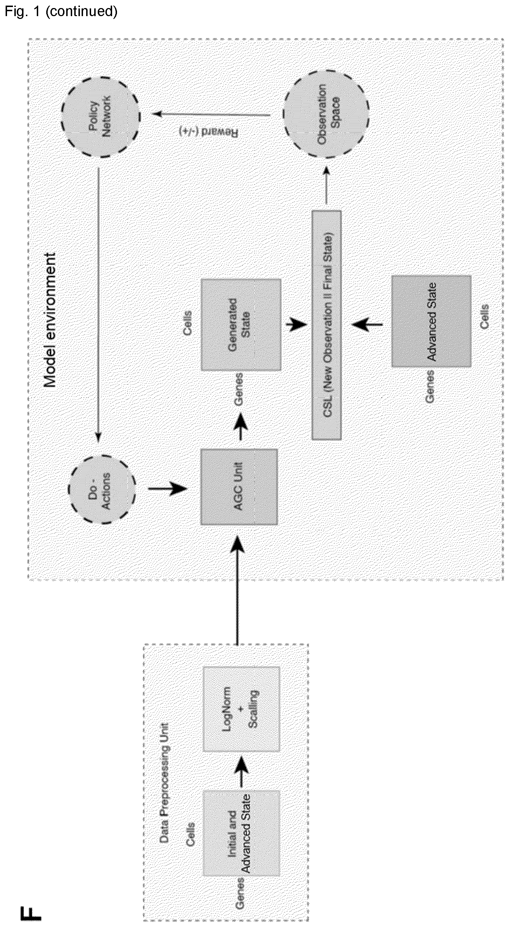

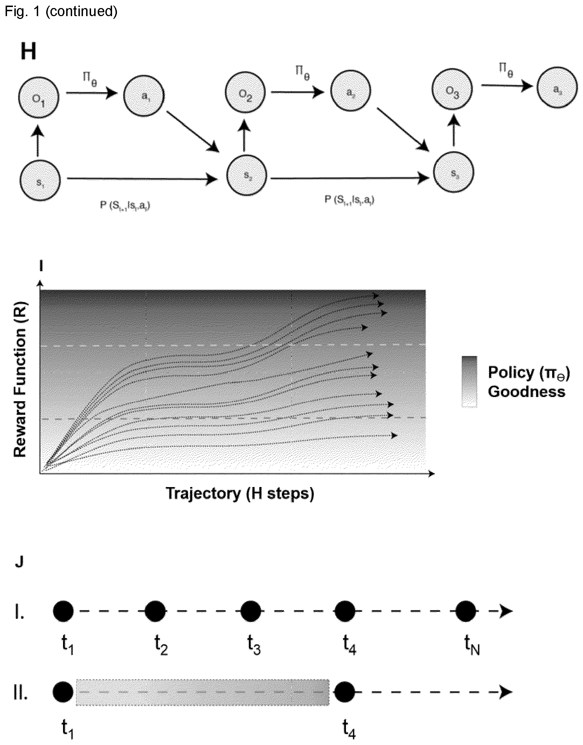

Fig. 1F: shows a schematic representation of a single-cell RNA-seq data preprocessing unit and model environment architecture. The left side represents the data preprocessing unit where the initial and corresponding advanced single-cell RNA-seq data are introduced and perform normalization and scaling steps. The preprocessed data is then introduced to the model environment. The model environment is composed of an AGC unit where the model can generate novel cellular states, interacting with the action unit (Do-Unit) that governs the generation process (Gen-Unit). The novel generated state is introduced to the CSL unit to calculate estimated rewards, which are then pushed to the policy network, aiming to learn optimal policies to find the right sequence of actions to maximize overall rewards;

Fig. 1G: shows a schematic representation of the causal paths that regulate underlying disease state trajectories over our lifetime;

Fig. 1H: shows a schematic representation of the Markov Decision Process where st represents the model environment state, Ot represents the observation space state, and at represents the action taken at time point t. $\pi_\Theta$ represents the policy network. P denotes the transition probability from the current states st to the next state $s_{t+1}$ after the model takes a certain action;

Fig. 1I: shows a schematic representation depicting the relationship between trajectory steps (H) (x-axis) and the reward (R) function (y-axis). The gray color gradient represents the policy ($\pi_\Theta$) optimality scale. The grey and white broken lines indicate low and high rewards, respectively;

Fig. 1J: shows a schematic representation of a two-state challenge, where data for two states of an evolving biological process data (at $t_1$ and $t_4$) are introduced to the model to reconstruct the hidden intermediate states. In the real data, there is ground truth for the intermediate states at time points $t_2$ and ts, which are used to estimate the reconstruction accuracy of the model;

Fig. 2A: shows a uniform manifold approximation and projection for dimension reduction (UMAP) representation of the cell cycle cellular states: G1 (green dots), S phase (yellow dots), and G2/M phase (red dots);

Fig. 2B: shows a UMAP representation of the two-state challenge, indicating the G1 and G2/M phases provided to the model as initial and advanced state, respectively;

Fig. 2C: shows a UMAP representation of the co-embedding for cell cycle real and generated cellular states;

Fig. 2D: shows a heat map depicting correlation patterns between real and generated data in each Leiden cluster;

Fig. 2E: shows a density plot of UMAP representation illustrating co-localization patterns of cellular states within each cell cycle phase and the generated states overlaying the cellular state manifold;

Fig. 2F: shows a UMAP representation of the epithelial-mesenchymal transition process single-cell RNA-seq data across experimental time points;

Fig. 2G: shows a UMAP representation for the two-state challenge, presenting the model data from Day 0 and Day 3 cellular states;

Fig. 2H: shows a density plot of UMAP representation showing co-localization patterns of cellular states from Day 0, Day 3, and generated states overlaying the cellular state manifold;

Fig. 2t: shows a UMAP representation displaying co-embedding of real and generated data. Colors indicate real and generated data within each Leiden cluster;

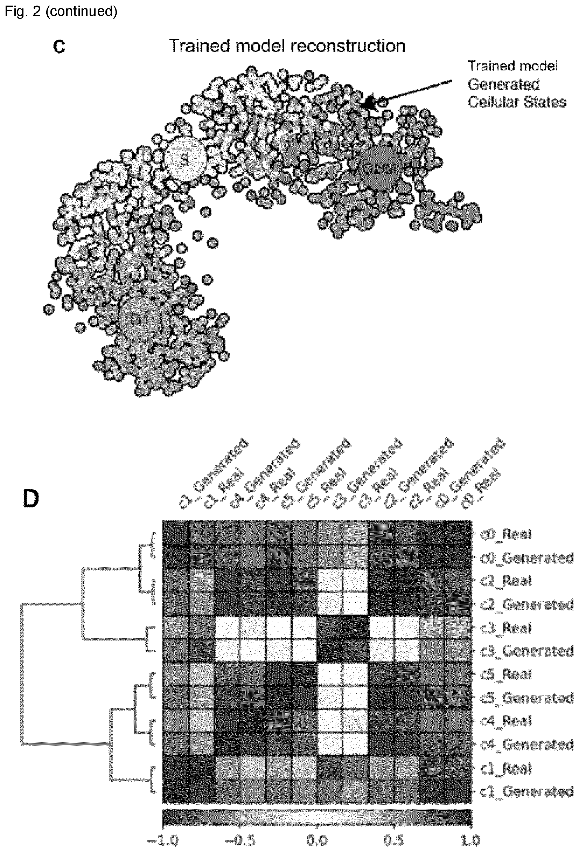

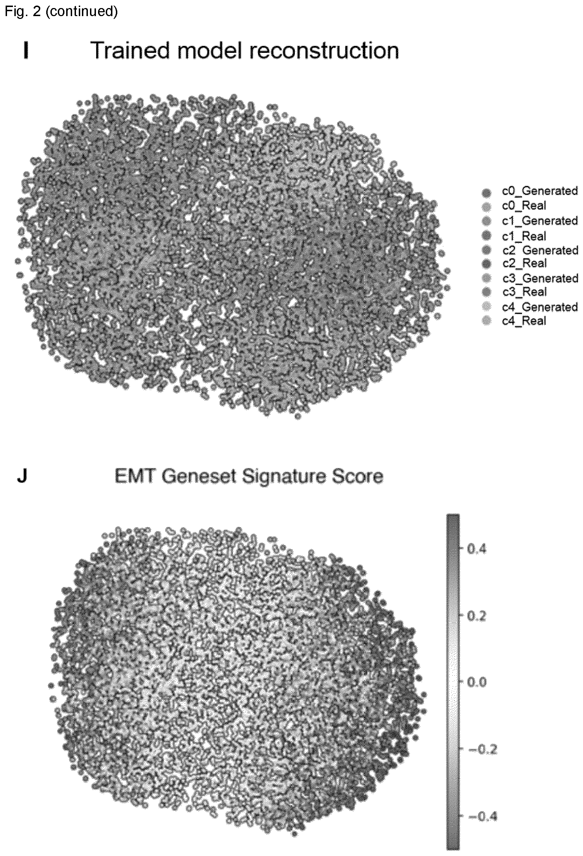

Fig. 2J: shows a UMAP representation displaying EMT gene signature scores during the transition process;

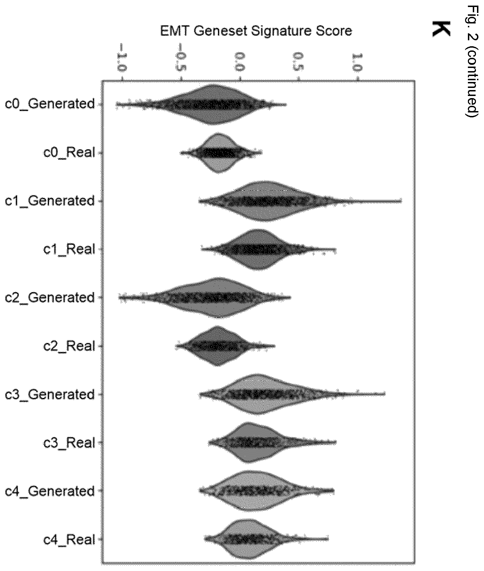

Fig. 2K: shows a violin plot depicting EMT scores of real and generated cellular states across the Leiden clusters;

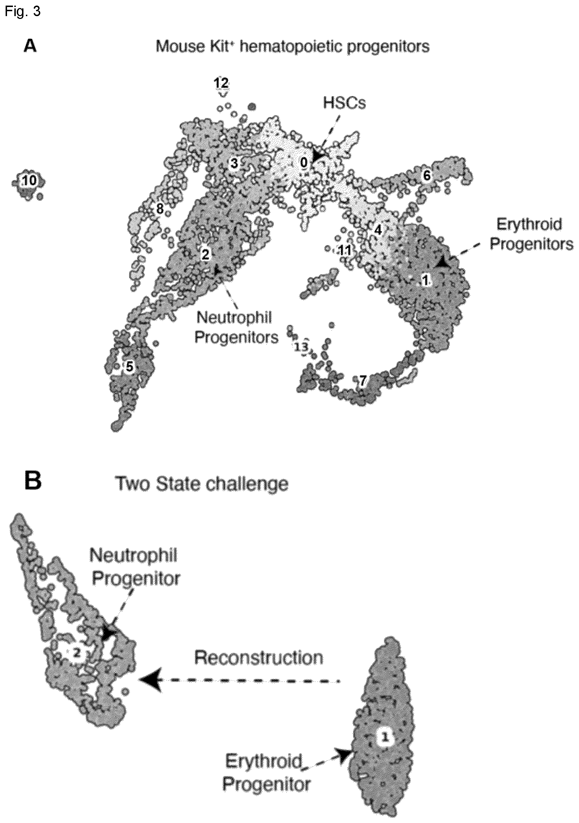

Fig. 3A: shows a UMAP representation of murine hematopoietic stem and progenitor single-cell RNA-seq data with cell type labels based on marker gene expression. Colors indicate Leiden clusters;

Fig. 3B: shows a UMAP representation for the two-state challenge, presenting neutrophil progenitors and erythroid progenitors to the model;

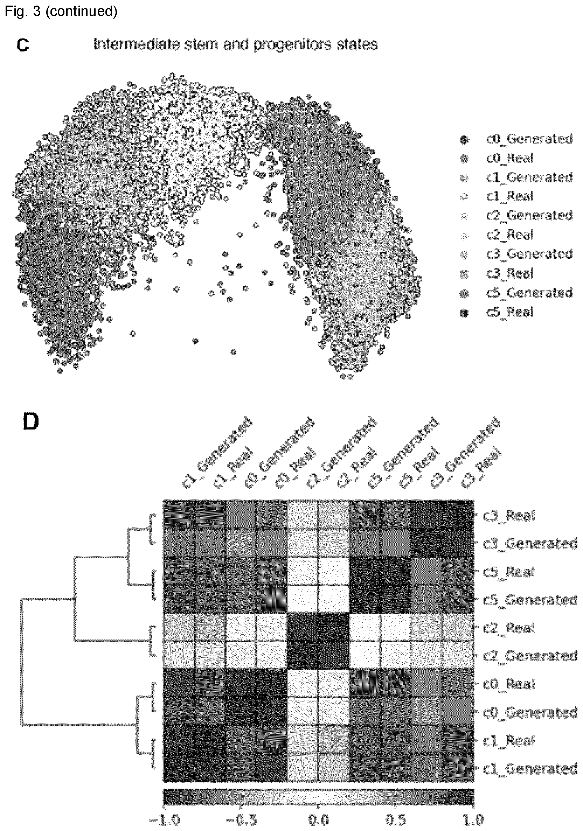

Fig. 3C: shows a UMAP representation displaying co-embedding of real and generated data with the Leiden clusters;

Fig. 3D: shows a heat map illustrating correlation patterns between real and generated data within each Leiden cluster;

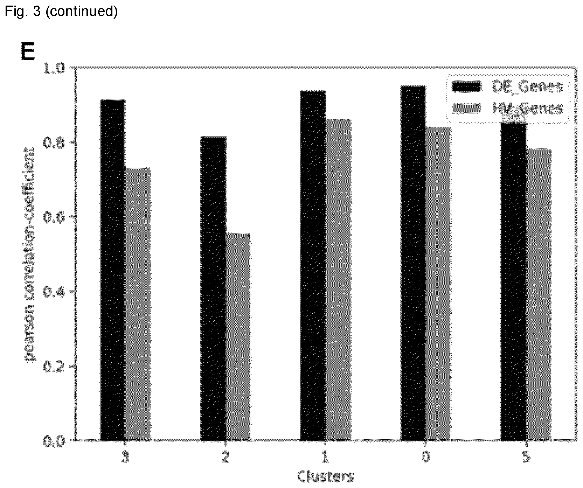

Fig. 3E: shows a barplot indicating Pearson's correlation coefficients between top 150 differentially expressed (DE) genes for real and generated data, and between highly variable genes (HVGs) within Leiden clusters;

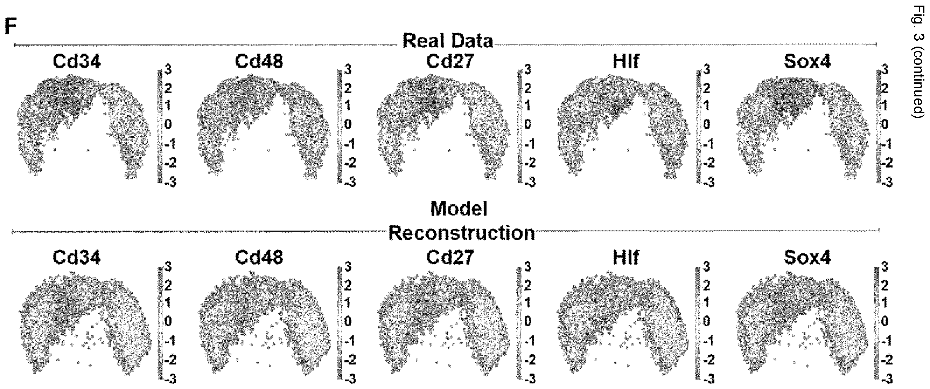

Fig. 3F: shows a UMAP representation of key hematopoietic stem cell (HSC) marker gene expression (normalized). The top panel represents real data gene expression, while the lower panel represents generated cellular states data;

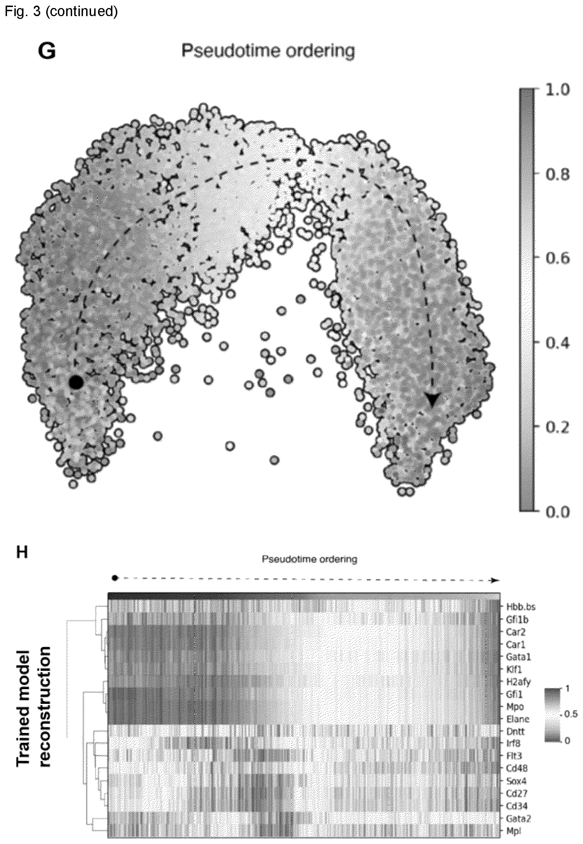

Fig. 3G: shows a UMAP representation of intermediate cellular states with a color code highlighting estimated pseudotime values;

Fig. 3H: shows a heat map displaying gene expression of key marker genes and transcription factors regulating HSCs and MMPs states across latent pseudotime of the model generated cellular states;

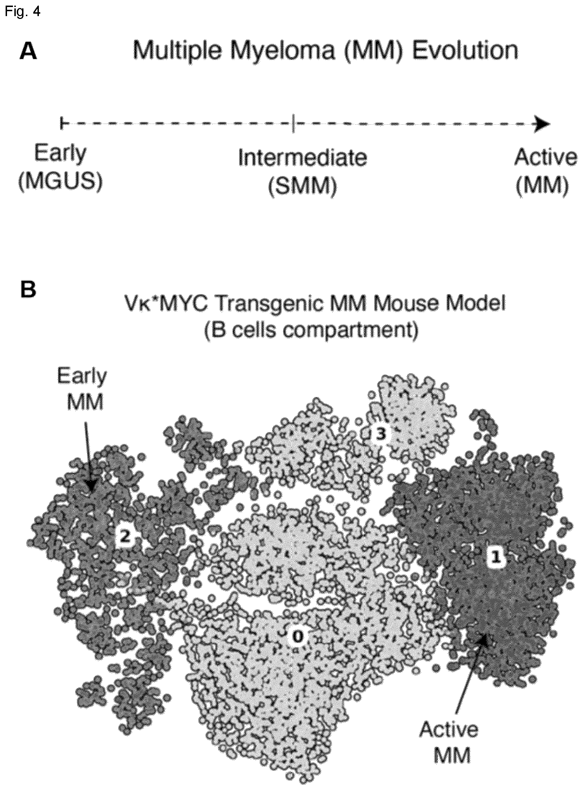

Fig. 4A: shows a diagram depicting the multistep evolutionary stages of MM tumors;

Fig. 4B: shows a UMAP representation of the B-cell compartment in the Vκ*MYC Transgenic MM Mouse Model, highlighting various stages of MM disease activity in Leiden clusters (MM, Intermediate, and Active MM);

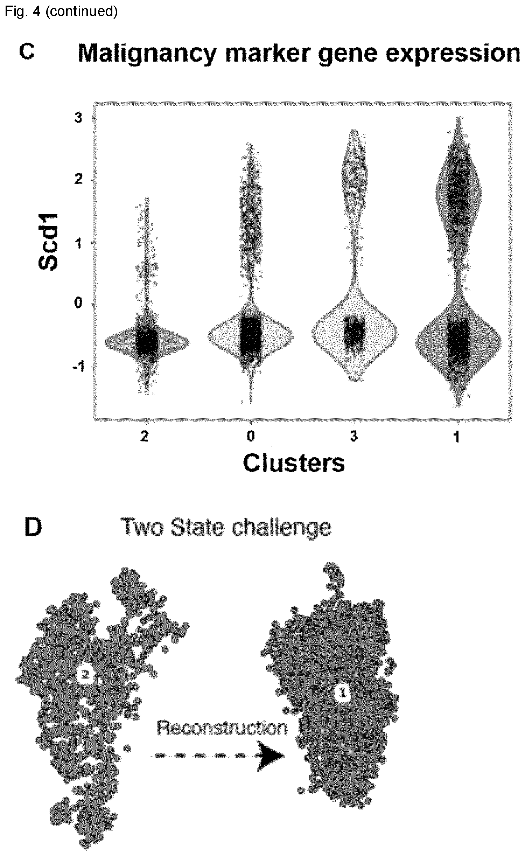

Fig. 4C: shows a violin plot displaying the normalized gene expression pattern of the Scd1 malignancy marker across different Leiden clusters, reflecting varying levels of MM disease activity (higher expression values indicate a more malignant state);

Fig. 4D: shows a UMAP representation for the two-state challenge, presenting early MM (Cluster 2) and active MM (Cluster 1) to the model;

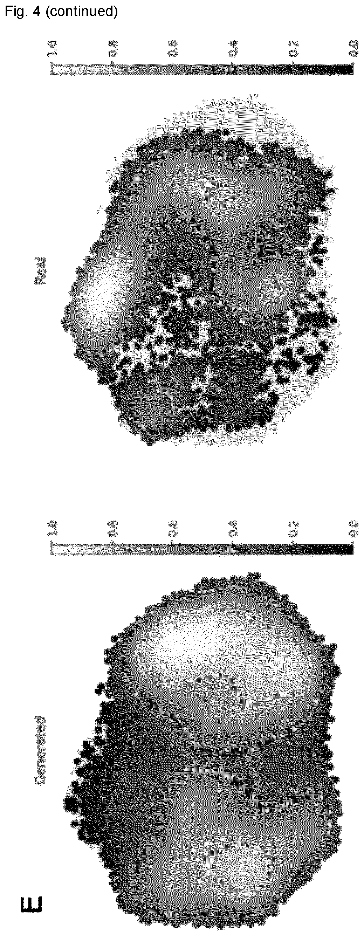

Fig. 4E: shows a density plot of UMAP representation demonstrating co-localization patterns of real and generated states overlaying the cellular states manifold;

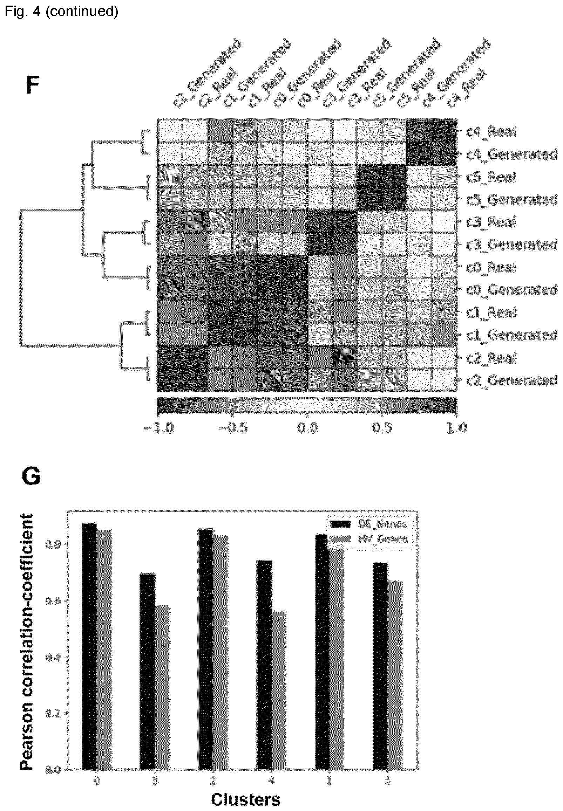

Fig. 4F: shows a heat map illustrating correlation patterns between gene expression of real and generated data in each Leiden cluster;

Fig. 4G: shows a bar plot indicating Pearson correlation coefficients between the top 150 differentially expressed (DE) genes for real and generated data, and between highly variable genes (HVGs) within Leiden clusters;

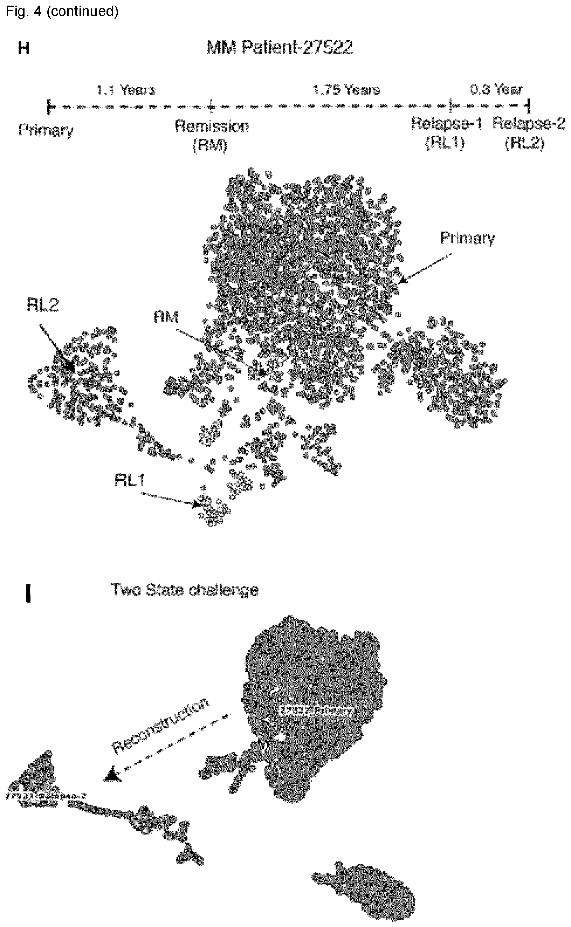

Fig. 4H: shows a UMAP representation of plasma cell sub clusters for Patient-27522 at Primary, Remission, Relapse-1, and Relapse-2 disease stages. Different colors represent distinct sub clusters within each time point;

Fig. 4I: shows a UMAP representation for the two-state challenge, presenting the Primary and Relapse-2 states to the model;

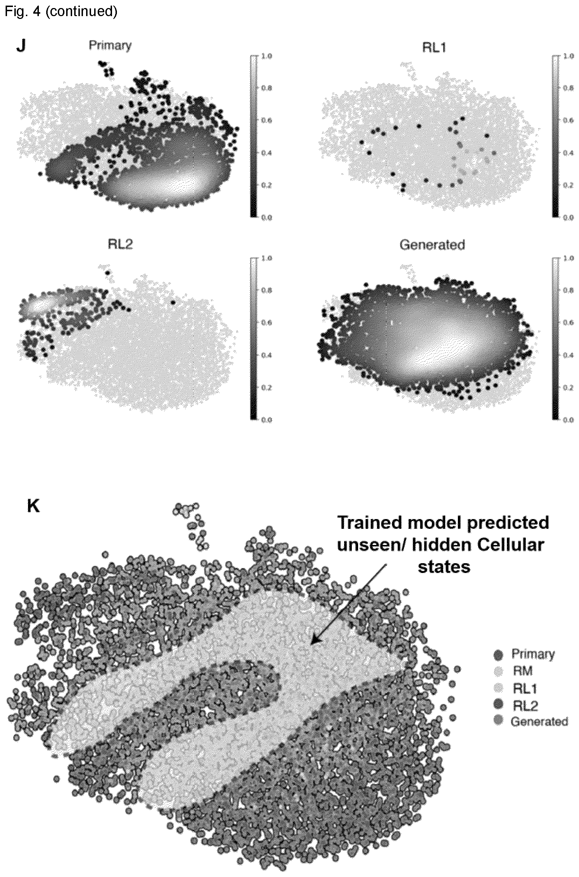

Fig. 4J: shows a density plot of UMAP representation displaying co-localization patterns of cellular states from Primary, Relapse-1, and Relapse-2, along with generated states overlaying the cellular state manifold;

Fig. 4K: shows a UMAP representation of co-embedding for real and generated MM cellular states. The highlighted dotted region represents the model predicted unseen/hidden cellular states, which are not present in the real data;

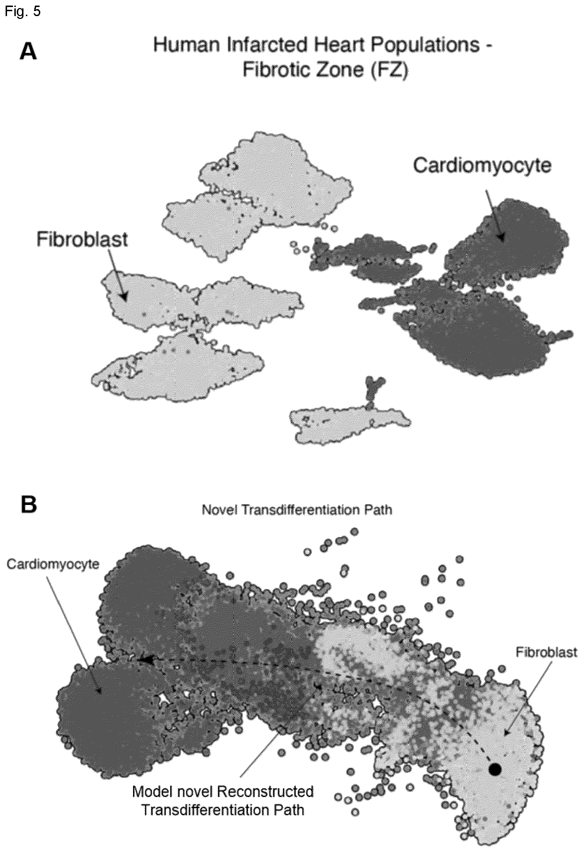

Fig. 5A: shows a UMAP representation of single-nucleus RNA-seq fibroblast and cardiomyocyte populations extracted from the Fibrotic Zone (FZ) of infarcted heart patients.

Colors indicate different cell identities;

Fig. 5B: UMAP representations illustrating the model novel reconstructed transdifferentiation path between fibroblasts and cardiomyocytes;

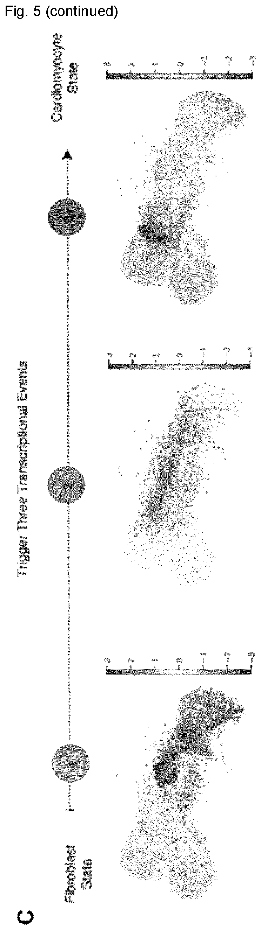

Fig. 5C: shows a UMAP representation displaying the key three transcriptional events regulating the transdifferentiation path and direct reprogramming of fibroblast states to cardiomyocyte states; and

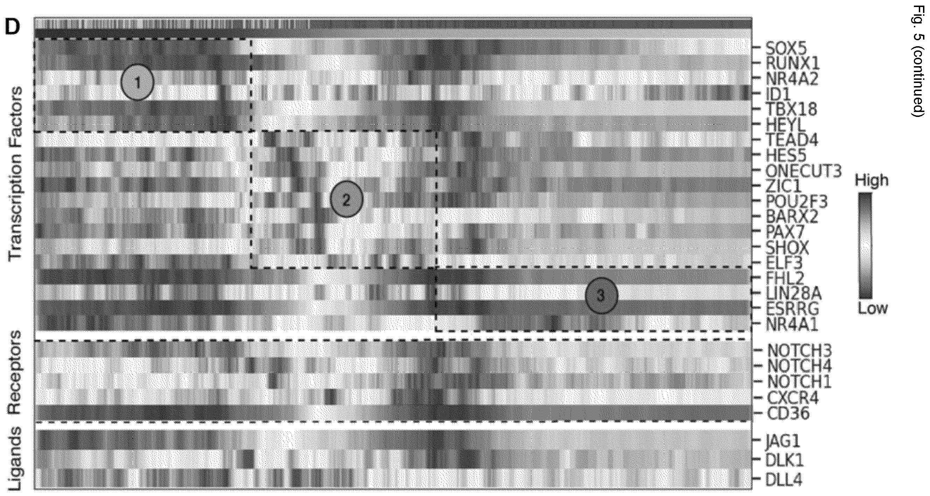

Fig. 5D: shows a heat map depicting transcription factors, ligands, and receptors regulating the three key transcriptional events governing the transdifferentiation path.

DETAILED DESCRIPTION OF EMBODIMENTS

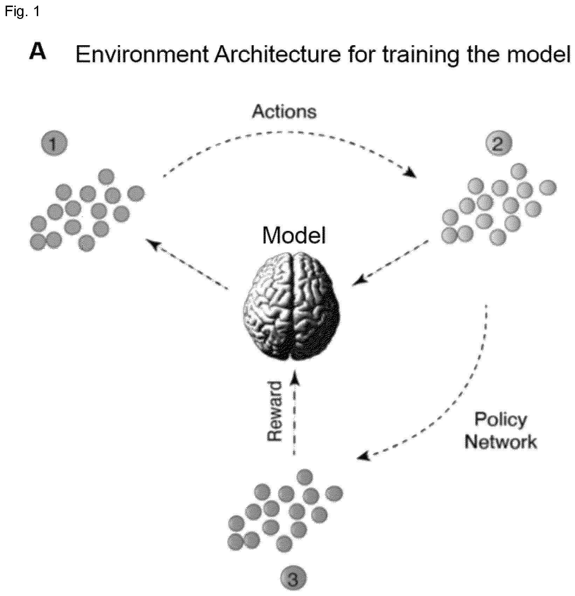

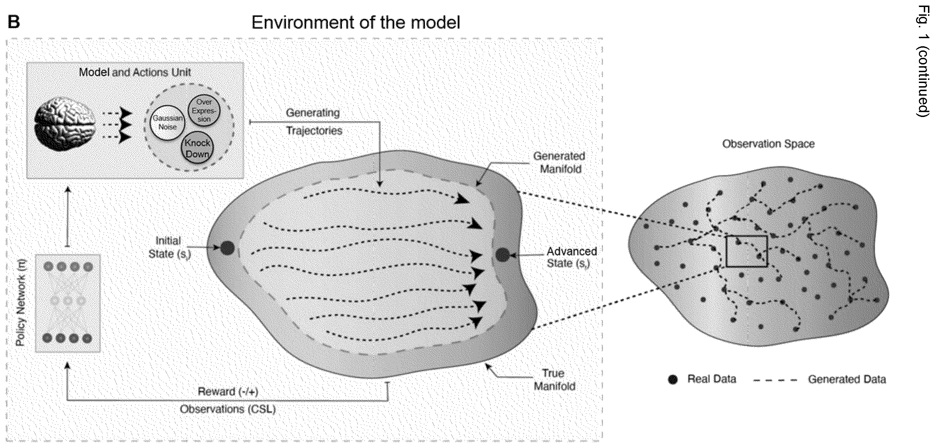

Figs. 1A, B and F show a deep reinforcement learning architecture of a model that is configured to reconstruct intermediate transcriptomic states on cellular differentiation trajectories connecting two anchoring states profiled by scRNA-seq.

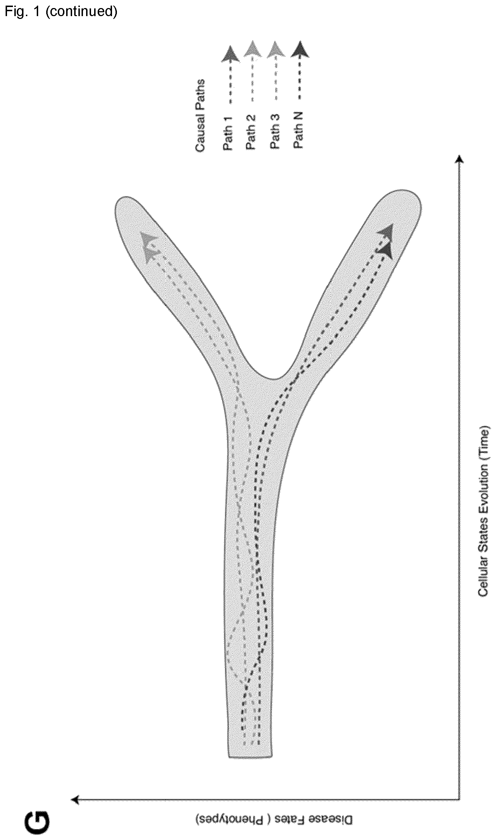

The model environment is composed of a model which can observe cellular states, take actions to generate new cellular states, and observe the consequences of taking such actions. The model environment emits a reward signal given the cellular state distance between the generated and advanced states. If the generated state is closer to the advanced state, the model receives positive reward to reinforce taking such actions consequently in future steps. Otherwise, if the generated state is far away from the advanced state, the model receives a negative reward to decrease the probability of taking such actions in future steps. In this way, the model may experience the environment over hundreds of thousands of steps, in order to learn how to generate the right sequence of actions for reconstructing causal cell state trajectories consistent with the permissible cell state manifold as depicted exemplary in Fig. 1G.

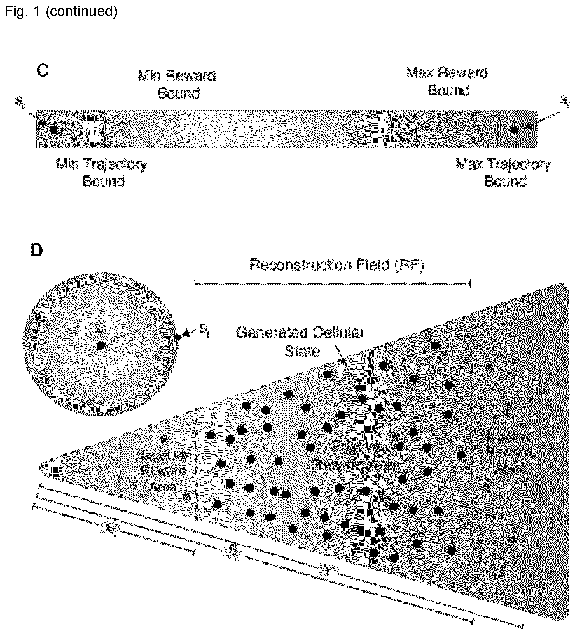

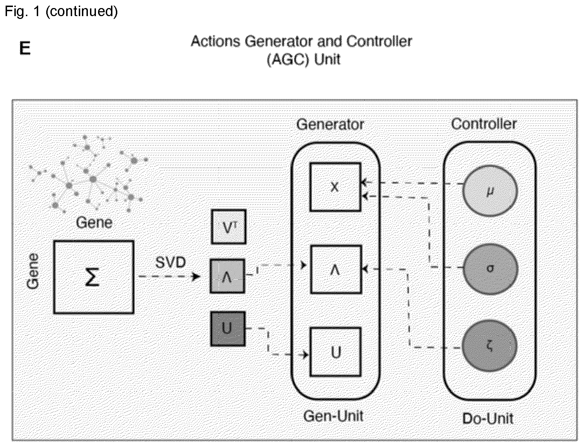

The input to model may be a merged single-cell gene expression matrix of the two anchoring cellular states (**S**, **Sf**), i.e., an initial cellular state and a corresponding advanced cellular state. The merged matrix may be pushed to the data processing unit and prepared for the model environment (Fig. 1F). To design reasoning models on a cell state manifold, several components may be incorporated into the model architecture: (1) The model environment may be designed as a Markov decision process (MDP) (Fig. 1H), where it is implicitly assumed that a cell fate only depends on the current cellular state, regardless of the entire cell state history. (2) The model observation space is defined as the manifold of all possible cell states which can be visited by the model (Fig. 1B). (3) The reconstruction field (RF) is constrained by distances to the anchoring cell states in the observation space. It guides reconstruction of the relevant region within the cell state manifold by the model (Fig. 1C, D). (4) The Actions Generator and Controller (AGC) unit enables the model to reconstruct cell states and to navigate in observation space (Fig. 1B). The AGC unit is composed of two subunits, termed Gen-Unit and Do-Unit (Fig. 1E). To harness the power of linear models for interpretable cell state reconstruction, the AGC unit may calculate a covariance matrix of the merged expression matrix of the two anchoring cell states.

This covariance matrix is decomposed into principal components (PC) by singular value decomposition (SVD). These PCs capture the gene-gene covariance structure and are utilized to guide the generation process within the Gen-Unit. The Gen-Unit can generate new cellular states by sampling from a multivariate Gaussian distribution, modeling changes of the individual PCs, where three control parameters ($\mu_{gen}$, $\sigma_{gen}$ and Q are governed by the Do-Unit. These actions can generate novel cellular states in gene expression space which respect the gene-gene covariance structure inherent in the anchoring cell states, aiming to confine the observation space to the actual cell state manifold. Hence, the Do-unit can enable the model to control the Gen-Unit during a learning process. (5) The reward function may be configured to be dependent on the cellular state loss (CSL), which quantifies the similarity of the generated cellular state to the advanced cellular state based on Kullback-Leibler divergence, cosine similarity and entropy regularization terms, and to satisfy the learning bound score (LBS) objective function (Methods, Figs. 1C, D). The reward function can reinforce the model to reconstruct cellular events in the reconstruction field of interest. (6) The model policy $\pi_{\Theta}$ can be parametrized by a neural network which is optimized via gradient ascent to maximize the reward objective (Fig. 1I). In addition to the standard reward objective, a maximum entropy objective can be configured to enable the model to alternate between exploration and exploitation phases during reconstruction. The model can generate stochastic policies by learning a probability distribution over actions given the collected observations generated by the AGC unit.

As demonstrated inter alia for the examples explained below, the model can be used for reconstructing unseen intermediate cell states in scenarios of varying complexity. In each case, it is started with a known continuous cell state trajectory profiled by scRNA-seq as ground truths, but only an initial and a corresponding advanced anchoring cell state is fed into the model to reconstruct the unseen intermediate states. Thereby, the model's capacity to reconstruct hidden states can be shown by directly comparing them to the unseen ground truths states (Fig. 1J). The challenges for the model span the reconstruction of linear trajectories, the reconstruction of multipotent states, as well as complex disease scenarios. The model has shown robustness of intermediate state reconstruction at the level of cell embeddings and across genes levels.

Further aspects and advantages of the invention result from the subsequent description of preferred examples.

EXAMPLES

Example 1: Reconstruction of the cell cycle

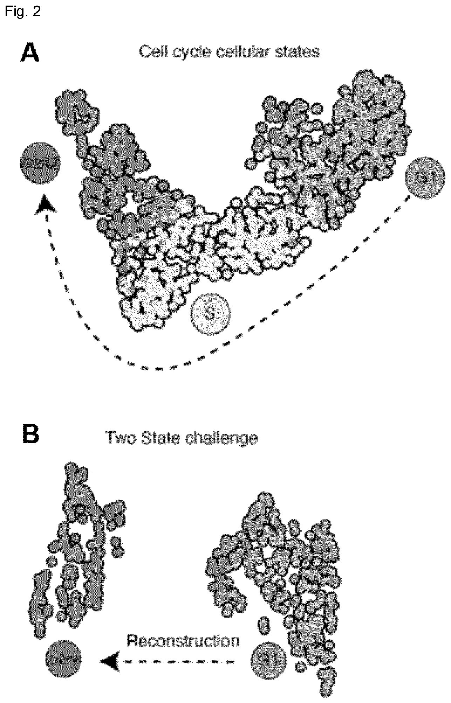

Murine hematopoietic progenitor cells going through the Interphase stage of the cell cycle were analysed (Fig. 2A).

The trained model has been employed by presenting G1 and G2 single cell RNAseq data to reconstruct the S phase (Fig. 2B).

During the training phase, the model went through an exploration phase followed by an exploitation phase, and ultimately led to reconstruction of the cellular states in the reconstruction field (RF) of interest. After training, the model was able to generate novel cellular states, successfully reconstructing the intermediate S phase, as well as G1 and G2 phases (Fig. 2C).

These results show that with the method according to the invention, unseen cellular states can be reconstructed.

Example 2: Reconstruction of shifts in cellular identity

The trained model can also be used to reconstruct an entire biological process involving shifts in cell identities.

Exemplary, it was chosen to investigate the epithelial-mesenchymal transition (EMT) process, which involves a transformation of a cell identity from epithelial to mesenchymal cellular states.

EMT is a complex process regulated by several key signalling pathways, including Wnt signalling pathways and transforming growth factor beta (TGF-$\beta$), which are crucial for cancer progression in metastatic conditions.

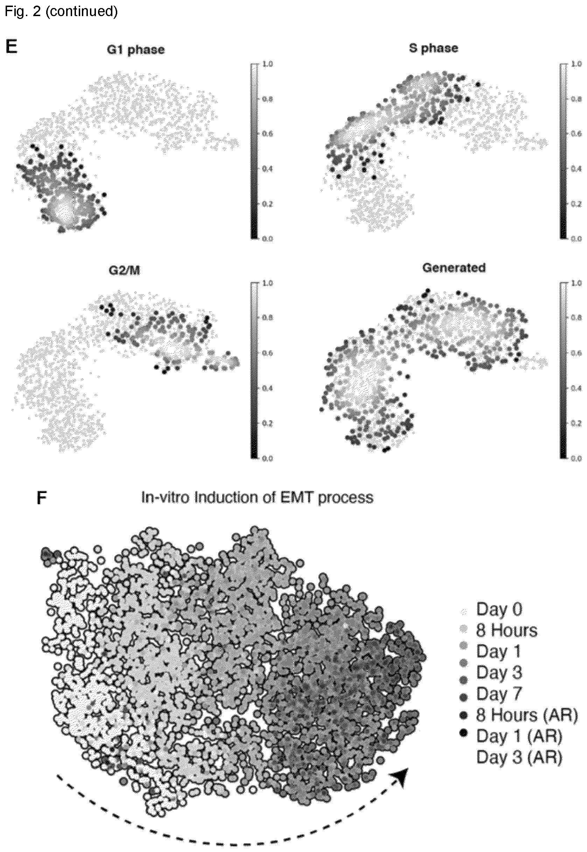

To study the temporal dynamics of the EMT process, single-cell RNA-seq data from Lung cancer cell-line (A549) treated with EMT-inducing factor (TGFB1) over a period of seven days were analysed (Fig. 2E).

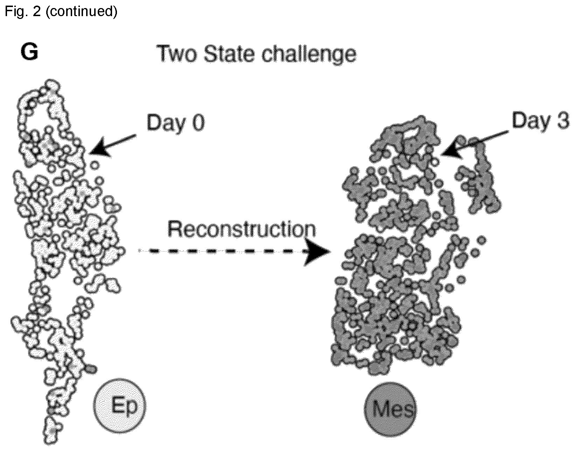

The model was used to introduce the single-cell RNA-seq data of only Day 0 and Day 3 to reconstruct the intermediate states (Fig. 2F). After training the model, the model was able to reconstruct the entire manifold (H= 10 steps, S2G and H) including 8 hours, 1 day and 7 days cellular states (Fig. 2G).

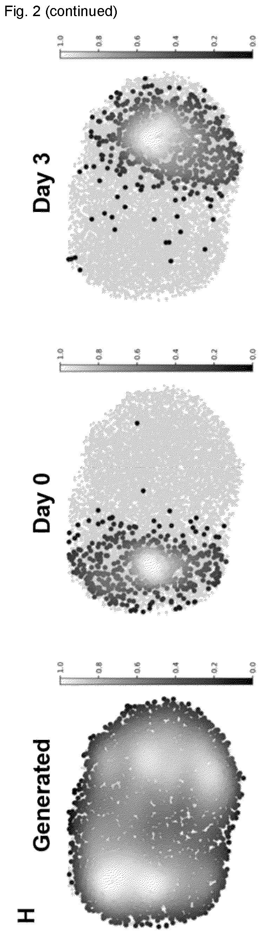

Moreover, the model successfully reconstructed the cellular states events at both the 2D embedding level of the real and generated sub-clusters (Fig. 2H) as well as key EMT maker genes.

To assess the quality of the generated data in reconstructing the EMT process, the EMT signature score was calculated. It could be observed that the generated cellular states scores distribution was similar to the real EMT scores distribution, which suggests that the model was able to reconstruct the entire EMT process accurately (Fig. 2J).

These findings demonstrate that with the method according to the invention, complex biological processes involving shifts in cell identity can be reconstructed, which provides potential applications in disease modelling.

Example 3: Reconstructing intermediate states in hematopoiesis

To test the ability of the model to accurately reconstruct complex intermediate states, the model was subjected to a more intricate biological process, such as hematopoiesis. This complex process takes place in the bone marrow and involves the gradual differentiation of hematopoietic stem cells (HSCs) into various types of blood cells, including red blood cells, platelets, and various types of white blood cells such as lymphocytes, neutrophils, and monocytes. The process is characterized by a continuous hierarchy of cellular transcriptional states, as HSCs progress through differentiation to multipotent progenitors (MPPs) and eventually commit to a single lineage.

To investigate the hematopoiesis process, single-cell RNA-seq data from Kit+ hematopoietic progenitors at the early stages of the hematopoietic hierarchy were utilized (Fig. 3A). The HSCs population and various haematopoietic progenitor cellular states were identified and the model was challenged by presenting two progenitor states, namely the erythroid and neutrophil progenitors, and tasked the model with reconstructing the stem cell and earlier progenitors' populations (Fig. 2B).

The model was capable of accurately reconstructing the entire early haematopoietic hierarchy including the stem and progenitors' states.

The accuracy and quality of the intermediate stem and progenitors' states reconstructed by the model was assessed by performing Leiden clustering and analysing co-embedding and gene expression correlation between the real and generated data. Nine clusters and selected the intermediate cluster subset (1,0; 2,3 and 5 cluster) for further analyses were defined.

It was possible to observe co-embedding alignment between real and generated data as well as highly correlation coefficient between real and generated data at the genes high dimensional space. The model was able to accurately reconstruct differentially expressed genes (n=150) and highly variable genes (n=3000), with up to 0.96 and 0.85 accuracy, respectively.

Moreover, the model was able to reconstruct co-expression patterns of HSCs markers (Cd34, Cd48, Hlf, Procr and Slamf1, Megakaryocyte progenitors (Mpl), erythroid progenitors' transcriptional factors (Gata1 and Gata2) and other progenitors marker genes (Fig. 2F).

Pseudotime inference for both real and generated data manifold was performed and pseudotime ordered the cells and key marker genes and transcriptional factors of the hematopoiesis process were examined (Figs. 3G, 3H). For example, it was possible to observe upregulation of (Elane) expression together with (Gfi1 and Mpo) maker genes. Then, the upregulation of the co-expression pattern of the HSCs markers was observed (CD48, SOX4, CD27 and CD34). In addition, the exclusive expression pattern of Gata2 and Gata1 was observed, which ultimately ended with the expression of CAR1, CAR2 and GFI1B.

These results show that the model was able to accurately identify the correct sequence of transcriptional events underlying the hematopoiesis process. The generative capabilities of the model allow for the generation of multiple realizations of connecting causal paths.

Example 4: Reconstructing Multiple Myeloma Intermediate States

The investigation with the model was expanded to further scenarios, such as cancer evolution, focusing on multiple myeloma (MM) disease evolution. MM is a bone marrow malignancy characterized by a multi-step process driven by genetic mechanisms and mutations, transforming the normal plasma cells through stages of increasing malignancy, including monoclonal gammopathy of undetermined significance (MGUS), smoldering myeloma (SMM), and ultimately to a high-risk malignant state with myeloma and plasma cell leukemia predominance.

To assess the model's ability to reconstruct intermediate MM states, single-cell RNAseq data from a Vκ*MYC transgenic mouse model were analysed. These mice exhibit a progressive accumulation of clonal plasma cells in the bone marrow, mirroring the evolutionary trajectory of human MM (Fig. 4A). By sub-setting the B-cell compartment and performing clustering, four major clusters representing different disease states were identified, ranging from early to intermediate and active disease stages (Fig. 4B). The malignancy marker Scd1 displayed a pattern of high expression that correlated with disease activity, with cluster 2 exhibiting low expression and cluster 1 showing the highest expression (Fig. 4C).

To train the model, the data from early MM (Cluster 2) and late MM (Cluster 1) were utilized as input.

The model accurately reconstructed the entire spectrum of MM disease states, including the intermediate states (Fig. 4E). Furthermore, alignment in the co-embedding of real and generated data (Fig. 4E) and high correlation coefficients in the high-dimensional gene expression space between the real and generated data was observed (Figs. 4F and 4G). These results demonstrate the capacity of the model in predicting the intermediate transformation processes during the tumorigenesis evolutionary steps.

Example 5: Reconstructing the disease progression

The malignant plasma cell compartment of a MM patient (MM-27522) who underwent remission (RM), relapse-1 (RL1), and relapse-2 (RL2) after the initial diagnosis (Primary) over a period of more than three years were analysed (Fig. 4H). The primary state and RL2 data and presented them to the model were extracted (Fig. 4J). Following the training phase, the model successfully reconstructed the entire disease progression with high accuracy (Fig. 4I). Notably, the model demonstrated the ability to predict unseen or hidden cellular states that were not previously observed in the data (Fig. 4K).

These results show that the model is able to accurately reconstruct the progression of an individual patients' disease, paving the way for novel personalized medicine and targeted therapy approaches.

Example 6: Predicting a novel path for fibroblasts-to-cardiomyocytes transdifferentiation

Myocardial infarction (MI) results in functional loss of cardiac muscle cells and impaired overall heart function. Following the occurrence of an infarction event, remodelling processes are initiated, characterized by an extensive inflammatory response. This response leads to the mobilization of innate and adaptive immune cells, notably macrophages, while the damaged tissue undergoes replacement by populations of fibroblasts. To address this issue, inducing fibroblasts-to-cardiomyocytes transdifferentiation has emerged as a potential approach to convert fibroblasts into functional cardiomyocytes, which has been tested in human and mouse. However, the underlying mechanism governing this process has remained elusive due to the lack of a systematic approach for predicting such transdifferentiating cellular states and the underlying molecular events.

Fibroblast and cardiomyocyte populations single-cell RNA-seq data from the fibrotic zone of MI patients' damaged heart tissue were utilized (Fig. 5A). Through training the model (S5K and S5M) on that two states data, it was possible to predict a novel fibroblasts-to-cardiomyocytes transdifferentiation path (Fig. 5B). This path is characterized by three key transcriptional events (Fig. 5C). Pseudotime inference and differential expression (DE) analysis was conducted, followed by pseudotime ordering of the DE genes based on the inferred pseudotime component.

The findings reveal the presence of three distinct modules that govern the predicted transdifferentiation path. The modules are composed of transcription factors (TFs), receptors (S5L), and ligands (Fig. 5D).

In the first event, upregulation of transcription factors (TFs) such as SOX5, RUNX1, NR4A2, and HEYL was observed (Event 1). This was followed by another event characterized by the upregulation of TEAD4, HES5, ONECUT3, ZIC1, POU2F3, BARX2, PAX7, SHOX, and ELF3 (Event 2). To ultimately commit to the cardiac fate lineage, the transdifferentiation path showed upregulation of FHL2, ESRRG, and NR4A1 (Event 3), along with the previously known GATA4, MEF2C, and TBX5 (GMT) reprogramming factors.

The results shed light on the molecular events driving fibro-to-cardiac transdifferentiation, providing insights into potential targets for therapeutic interventions. Based on these results enhancing cardiac regeneration and improving heart function in MI patients may be achieved.

It shall be understood that a preferred embodiment of the invention can also be any combination of the dependent claims or above embodiments with the respective independent claim.

In the claims, the word "comprising" does not exclude other elements or steps, and the indefinite article "a" or "an" does not exclude a plurality.

A single unit or device may fulfil the functions of several items recited in the claims. The mere fact that certain measures are recited in mutually different dependent claims does not indicate that a combination of these measures cannot be used to advantage.

Procedures like providing transcriptome data, providing a model trained by a machine learning and generating the at least one intermediate cellular state, etc. performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any units described herein may be processing units that are part of a classical computing system. Processing units may include a general-purpose processor and may also include a field programmable gate array (FPGA), an application specific integrated circuit (ASIC), or any other specialized circuit. Any memory may be a physical system memory, which may be volatile, non-volatile, or some combination of the two. The term "memory" may include any computer-readable storage media such as a non-volatile mass storage. If the computing system is distributed, the processing and/or memory capability may be distributed as well. The computing system may include multiple structures as "executable components". The term "executable component" is a structure well understood in the field of computing as being a structure that can be software, hardware, or a combination thereof. For instance, when implemented in software, one of ordinary skill in the art would understand that the structure of an executable component may include software objects, routines, methods, and so forth, that may be executed on the computing system. This may include both an executable component in the heap of a computing system, or on computer-readable storage media. The structure of the executable component may exist on a computer-readable medium such that, when interpreted by one or more processors of a computing system, e.g., by a processor thread, the computing system is caused to perform a function. Such structure may be computer readable directly by the processors, for instance, as is the case if the executable component were binary, or it may be structured to be interpretable and/or compiled, for instance, whether in a single stage or in multiple stages, so as to generate such binary that is directly interpretable by the processors. In other instances, structures may be hard coded or hard wired logic gates, that are implemented exclusively or near-exclusively in hardware, such as within a field programmable gate array (FPGA), an application specific integrated circuit (ASIC), or any other specialized circuit. Accordingly, the term "executable component" is a term for a structure that is well understood by those of ordinary skill in the art of computing, whether implemented in software, hardware, or a combination. Any embodiments herein are described with reference to acts that are performed by one or more processing units of the computing system. If such acts are implemented in software, one or more processors direct the operation of the computing system in response to having executed computer-executable instructions that constitute an executable component. Computing system may also contain communication channels that allow the computing system to communicate with other computing systems over, for example, network. A "network" is defined as one or more data links that enable the transport of electronic data between computing systems and/or modules and/or other electronic devices. When information is transferred or provided over a network or another communications connection, for ex-ample, either hardwired, wireless, or a combination of hardwired or wireless, to a computing system, the computing system properly views the connection as a transmission medium. Transmission media can include a network and/or data links which can be used to carry desired program code means in the form of computer-executable instructions or data structures and which can be accessed by a general-purpose or special-purpose computing system or combinations. While not all computing systems require a user interface, in some embodiments, the computing system includes a user interface system for use in interfacing with a user. User interfaces act as input or output mechanism to users for instance via displays.

Those skilled in the art will appreciate that at least parts of the invention may be practiced in network computing environments with many types of computing system configurations, including, personal computers, desktop computers, laptop computers, message processors, hand-held devices, multi-processor systems, microprocessor-based or programmable consumer electronics, network PCs, minicomputers, main-frame computers, mobile telephones, PDAs, pagers, routers, switches, data-centres, wearables, such as glasses, and the like. The invention may also be practiced in distributed system environments where local and remote computing system, which are linked, for example, either by hardwired data links, wireless data links, or by a combination of hardwired and wireless data links, through a network, both perform tasks. In a distributed system environment, program modules may be located in both local and remote memory storage devices.

Those skilled in the art will also appreciate that at least parts of the invention may be practiced in a cloud computing environment. Cloud computing environments may be distributed, although this is not required. When distributed, cloud computing environments may be distributed internationally within an organization and/or have components possessed across multiple organizations. In this description and the following claims, "cloud computing" is defined as a model for enabling on-demand network access to a shared pool of configurable computing resources, e.g., networks, servers, storage, applications, and services. The definition of "cloud computing" is not limited to any of the other numerous advantages that can be obtained from such a model when deployed. The computing systems of the figures include various components or functional blocks that may implement the various embodiments disclosed herein as explained. The various components or functional blocks may be implemented on a local computing system or may be implemented on a distributed computing system that includes elements resident in the cloud or that implement aspects of cloud computing. The various components or functional blocks may be implemented as software, hardware, or a combination of software and hardware. The computing systems shown in the figures may include more or less than the components illustrated in the figures and some of the components may be combined as circumstances warrant.

Any reference signs in the claims should not be construed as limiting the scope.

**Claims**

1. A method for generating at least one intermediate cellular state of a cellular state evolution from an initial cellular state to a corresponding advanced cellular state, the method comprising the steps of:

   - providing transcriptome data representing the initial cellular state and the corresponding advanced cellular state,
   - providing a model trained by a machine learning for generating the at least one intermediate cellular state on the basis of the initial cellular state and the corresponding advanced cellular state, and
   - generating the at least one intermediate cellular state using the provided trained model on the basis of the initial cellular state and the corresponding advanced cellular state.

2. The method according claim 1, wherein the transcriptome data representing the initial cellular state and the corresponding advanced cellular state are clinical data obtained experimentally from a patient.

3. The method according to any of the preceding claims, wherein the initial and advanced cellular state refer to

   - different phases of the cells in cell division,
   - different phases of the cells in cellular differentiation,
   - different phases of the cells in the transformation of cellular identity, particularly in cellular transdifferentiation,
   - different phases of the cells in the transformation of a cell to a cancer cell, or
   - different phases of the cells in cell the transformation of a cancer cell to a metastatic cancer cell.

4. The method according to any of the preceding claims, wherein based on the at least one intermediate cellular state, a causal trajectory for of the cellular state evolution from the initial cellular state to the corresponding advanced cellular state is generated.

5. The method according to any of the preceding claims, wherein the model is trained by a deep reinforcement learning technique.

6. A method for training a model by machine learning, the method comprising the steps of:

   i) providing a plurality of transcriptome training data, each representing an initial cellular state and a corresponding advanced cellular state of a cellular state evolution,
   ii) providing a model that can be trained by machine learning, and
   iii) training the model by machine learning using the transcriptome training data such that the trained model is configured to receive transcriptome data representing an initial cellular state and a corresponding advanced cellular state of a cellular state evolution and for generating at least one intermediate cellular state of the cellular state evolution from the initial cellular state to the corresponding advanced cellular state based on the received transcriptome data.

7. The method according claim 6, wherein the transcriptome training data, each representing the initial cellular state and the corresponding advanced cellular state, respectively, are clinical data obtained experimentally from one or more patients.

8. The method according to claim 6 or 7, wherein step iii) comprises at least one of the following steps:

   iii.1) generating transcriptome data of further cellular states by sampling from a multivariate Gaussian distribution to learn combinations of action parameters comprising a control mean and a standard deviation of the generated transcriptome data of transcriptome data of further cellular states,

   iii.2) classifying the generated transcriptome data of further cellular states based on the learned combinations of action parameters to identify those combinations of action parameters that fulfil a predefined reward criterion,

   iii.3) calculating a distance of the generated transcriptome data of further cellular states to the transcriptome data of advanced cellular states of the plurality of transcriptome training data,

   iii.4) rewarding the generated transcriptome data of further cellular states with reference to a predefined reward criterion, and

   iii.5) selecting a combination of action parameters to generate transcriptome data of further cellular states, which have the highest probability of an increasing reward from one generated transcriptome data to the next transcriptome data within the sequence, as the transcriptome data of a plurality of sequential intermediate cellular states.

9. The method according to any of claims 6 to 8, wherein the advanced cellular state in step i) is divided into a plurality of groups of different severity of the advanced cellular state,

   preferably wherein the transcriptome data of a plurality of sequential intermediate cellular states selected and generated in step iii.5) is assigned to one of the groups of different severity of the advanced cellular state.

10. The method according to any of the preceding claims, wherein step iii) further comprises

    iii.7) repeating the steps iii.1) to iii.5) to generate a plurality of transcriptome data of a plurality of sequential intermediate cellular states.

11. A method for diagnosing a disease, comprising

    a) providing a sample of a subject, wherein the sample comprises cells associated with the disease to be diagnosed,

    b) obtaining the transcriptome data of one or more cells from the sample obtained in step a), and

    c) comparing the transcriptome data obtained in step b) with the reconstructed transcriptome data of the plurality of sequential intermediate cellular states as generated in the method according to any of claims 1 to 5,

    wherein the disease is diagnosed if the transcriptome data obtained in step b) corresponds to one of the transcriptome data of the plurality of sequential intermediate cellular states.

12. The method according to claim 11, wherein in step c) the transcriptome data obtained in step b) is further compared with the transcriptome data of a plurality of cells in an advanced cellular state as provided in the method according to any of claims 1 to 5, and/or with the transcriptome data of a plurality of cells in an initial cellular state as provided in the method according to any of claims 1 to 5,

    wherein the disease is diagnosed if the transcriptome data obtained in step b) corresponds to one of the transcriptome data of the plurality of sequential intermediate cellular states or to one of the transcriptome data of the advanced cellular state,

    and/or

    wherein the disease is not diagnosed if the transcriptome data obtained in step b) corresponds to one of the transcriptome data of the initial cellular state.

13. A method for predicting disease progression, comprising

    A) providing a sample of a subject, wherein the sample comprises cells associated with the disease to be diagnosed,

    B) obtaining the transcriptome data of one or more cells from the sample obtained in step a), and

    C) comparing the transcriptome data obtained in step B) with the reconstructed transcriptome data of the at least one intermediate cellular state as generated in the method according to claims 1 to 5,
to identify one of the transcriptome data of the at least one intermediate cellular state as generated in the method according to claims 1 to 5, to which the transcriptome data obtained in step B) corresponds

wherein the prediction of the disease is based on the group of the severity of the disease, to which the transcriptome data identified in step C) is assigned.

14. The method according to any of claims 11 to 13, wherein the disease is cancer, preferably wherein the disease is metastasizing cancer.

15. A method for transforming the identity of a cell, the method comprising

1) generating at least one intermediate cellular state of a cellular state evolution from an initial cellular state to a corresponding advanced cellular state, with a method according to any of claims 1 to 5,
wherein the initial and advanced cellular state refer to different phases of the cells in the transformation of cellular identity, particularly in cellular transdifferentiation,
2) analysing the at least one intermediate cellular state generated in step 1) to identify a cellular signalling pathway involved in the transformation of the cellular identity,
3) modulating, preferably activating or inactivating, the cellular signalling pathway identified in step 2) to promote the transformation of the identity of a cell.

Fig. 1

**A** Environment Architecture for training the model

Fig. 1 (continued)

Fig. 1 (continued)

Fig. 1 (continued)

**E**

Actions Generator and Controller
(AGC) Unit

Fig. 1 (continued)

Fig. 1 (continued)

Causal Paths

Path 1
Path 2
Path 3
Path N

Cellular States Evolution (Time)

Disease Fates ( Phenotypes)

Fig. 1 (continued)

**H**

**I**

**Reward Function (R)**

**Trajectory (H steps)**

Policy ($\pi_\Theta$)
Goodness

**J**

I.

$t_1$    $t_2$    $t_3$    $t_4$    $t_N$

II.

$t_1$    $t_4$

Fig. 2

A    Cell cycle cellular states

B    Two State challenge

Fig. 2 (continued)

**C**  Trained model reconstruction

**D**

Fig. 2 (continued)

**E**

G1 phase

S phase

G2/M

Generated

**F** In-vitro Induction of EMT process

Day 0
8 Hours
Day 1
Day 3
Day 7
8 Hours (AR)
Day 1 (AR)
Day 3 (AR)

Fig. 2 (continued)

**G**   Two State challenge

Day 0

Reconstruction

Day 3

Ep

Mes

Fig. 2 (continued)

Fig. 2 (continued)

## I   Trained model reconstruction

- c0_Generated
- c0_Real
- c1_Generated
- c1_Real
- c2_Generated
- c2_Real
- c3_Generated
- c3_Real
- c4_Generated
- c4_Real

## J   EMT Geneset Signature Score

0.4

0.2

0.0

−0.2

−0.4

Fig. 2 (continued)

## K

Fig. 3

**A**    Mouse Kit⁺ hematopoietic progenitors

**B**    Two State challenge

Fig. 3 (continued)

**C**      Intermediate stem and progenitors states

- c0_Generated
- c0_Real
- c1_Generated
- c1_Real
- c2_Generated
- c2_Real
- c3_Generated
- c3_Real
- c5_Generated
- c5_Real

**D**

Fig. 3 (continued)

F

Real Data

Cd34   Cd48   Cd27   Hlf   Sox4

Model
Reconstruction

Cd34   Cd48   Cd27   Hlf   Sox4

Fig. 3 (continued)

EP 4 625 417 A1

Fig. 3 (continued)

**G** Pseudotime ordering

**H**

Pseudotime ordering

Trained model reconstruction

Fig. 4

**A**  Multiple Myeloma (MM) Evolution

Early (MGUS) — Intermediate (SMM) — Active (MM)

**B**  Vκ*MYC Transgenic MM Mouse Model
(B cells compartment)

Early MM

Active MM

Fig. 4 (continued)

## C Malignancy marker gene expression

## D Two State challenge

Fig. 4 (continued)

Fig. 4 (continued)

**F**

**G**

Fig. 4 (continued)

**H**                    MM Patient-27522

**I**     Two State challenge

Fig. 4 (continued)

Fig. 5

**A**

Human Infarcted Heart Populations -
Fibrotic Zone (FZ)

Cardiomyocyte

Fibroblast

**B**

Novel Transdifferentiation Path

Cardiomyocyte

Fibroblast

Model novel Reconstructed
Transdifferentiation Path

Fig. 5 (continued)

Fig. 5 (continued)

Europäisches
Patentamt

European
Patent Office

Office européen
des brevets

# EUROPEAN SEARCH REPORT

**Application Number**

EP 24 16 5826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/253070 A1 (DREWS JOSHUA [US] ET AL) 10 August 2023 (2023-08-10) | 1-3,5-15 | INV. G16B5/20 |
| Y | * whole document, particularly: claims 1, 14, 15 Fig 12A-F, Fig 13 [0137] [0774] [0776] [0834] [0887] [0894] [0929] * | 4 | G16B40/20 G16B10/00 |
| | ----- | | |
| Y | US 2023/268024 A1 (RIESENFELD SAMANTHA J [US] ET AL) 24 August 2023 (2023-08-24) | 4 | |
| A | * whole document, particularly claim 1, [0015] * | 1-3,5-15 | |
| | ----- | | |
| A | CN 116 259 364 A (UNIV HUNAN) 13 June 2023 (2023-06-13) * whole document, in particular [0002] * | 1-15 | |
| | ----- | | |
| A | UTHAMACUMARAN ABICUMARAN: "A review of dynamical systems approaches for the detection of chaotic attractors in cancer networks", PATTERNS, [Online] vol. 2, no. 4, 9 April 2021 (2021-04-09), page 100226, XP093113904, ISSN: 2666-3899, DOI: 10.1016/j.patter.2021.100226 Retrieved from the Internet: URL:https://www.sciencedirect.com/science/article/pii/S2666389921000404> [retrieved on 2024-09-09] * whole document, in particular: p.7, col. 1, par. 1-2 p.10, col.2, par. 3 * | 1-15 | **TECHNICAL FIELDS SEARCHED (IPC)** G16B |
| | ----- | | |
| | -/-- | | |

The present search report has been drawn up for all claims

| Place of search | Date of completion of the search | Examiner |
|---|---|---|
| Munich | 18 September 2024 | Eberhardt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 24 16 5826 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ABICUMARAN UTHAMACUMARAN ET AL: "A Review of Mathematical and Computational Methods in Cancer Dynamics", ARXIV.ORG, CORNELL UNIVERSITY LIBRARY, 201 OLIN LIBRARY CORNELL UNIVERSITY ITHACA, NY 14853, [Online] 5 January 2022 (2022-01-05), XP091303597, DOI: 10.3389/FONC.2022.850731 [retrieved on 2024-09-09] * the whole document * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 18 September 2024 | Eberhardt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 5826

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2024

| Patent document cited in search report | Publication date | Patent family member(s) | Publication date |
|---|---|---|---|
| US 2023253070 A1 | 10-08-2023 | NONE | |
| US 2023268024 A1 | 24-08-2023 | NONE | |
| CN 116259364 A | 13-06-2023 | NONE | |

EPO FORM P0459

For more details about this annex : see Official Journal of the European Patent Office, No. 12/82

## REFERENCES CITED IN THE DESCRIPTION

**Non-patent literature cited in the description**

- **SCHIEBINGER et al.** *Cell*, 2019
- **GORIN G et al.** *PLoS Comput Biol*, 2022
- **LOTFOLLAHI et al.** *Nat Methods*, 2019